(19) **Europäisches Patentamt / European Patent Office / Office européen des brevets**

(11) **EP 4 736 757 A2**

(12) # EUROPEAN PATENT APPLICATION

(43) Date of publication:
06.05.2026 Bulletin 2026/19

(21) Application number: 26167009.5

(22) Date of filing: 06.12.2022

(51) International Patent Classification (IPC):
*A61B 5/11* (2006.01)

(52) Cooperative Patent Classification (CPC):
**A61B 5/4839; A61B 5/0215; A61B 5/1107;**
**A61B 5/4875; A61B 5/686; A61B 5/7203;**
**A61B 5/7257; A61B 5/726; A61B 5/7267;**
A61B 2505/07; A61B 2505/09; A61B 2562/0233

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: 07.12.2021 US 202163287003 P
07.12.2021 US 202163287009 P
07.12.2021 US 202163287016 P
17.12.2021 US 202163291253 P
17.12.2021 US 202163291270 P

(62) Document number(s) of the earlier application(s) in
accordance with Art. 76 EPC:
**22854525.7 / 4 408 262**

(71) Applicant: **Edwards Lifesciences Corporation**
**Irvine, CA 92614 (US)**

(72) Inventor: **KEIDAR, Yaron**
**3079892 Caesarea (IL)**

(74) Representative: **Venner, Julia Ann et al**
**Kilburn & Strode LLP**
**Lacon London**
**84 Theobalds Road**
**London WC1X 8NL (GB)**

Remarks:
This application was filed on 24.03.2026 as a
divisional application to the application mentioned
under INID code 62.

(54) **LAP SIGNAL PROCESSING TO AUTOMATICALLY CALCULATE A/V RATIO**

(57) Systems and methods for cardiac monitoring and treatment using left atrial pressure are described herein. In many aspects, a pressure sensor implantable into the left atria, of a patient's heart records a pressure signal. The left atrial pressure can be used to calculate an A/V ratio, which has clinical significance, and in turn can be used to guide treatment and/or as part of an open or closed loop automated treatment system.

*FIG. 3A*

EP 4 736 757 A2

## Description

### CROSS-REFERENCE TO RELATED APPLICATIONS

The current application claims the benefit of and priority under 35 U.S.C. § 119(e) to U.S. Provisional Patent Application No. 63/287,003 entitled "LAP SIGNAL PROCESSING TO AUTOMATICALLY CALCULATE A/V RATIO" filed December 7, 2021, to U.S. Provisional Patent Application No. 63/287,009 entitled "EXERTION RESPONSE DETECTION BY AN IMPLANTABLE SENSOR" filed December 7, 2021, U.S. Provisional Patent Application No. 63/287,016 entitled "IMPROVED SIGNAL PROCESSING OF BLOOD PRESSURE FROM AN IMPLANTABLE SENSOR BY PRE-CLASSIFICATION OF PATIENTS" filed December 7, 2021, U.S. Provisional Patent Application No. 63/291,253 entitled "SYSTEMS AND METHODS FOR HEART VALVE MONITORING AND REPLACEMENT" filed December 17, 2021, and U.S. Provisional Patent Application No. 63/291,270 entitled "SYSTEMS AND METHODS FOR PUMONARY CONGESTION MONITORING AND TREATMENT" filed December 17, 2021. The disclosures of U.S. Provisional Patent Application Nos. 63/287,003, 63/287,009, 63/287,016, 63/291,253, and 63/291,270 are hereby incorporated by reference in their entireties for all purposes.

### FIELD OF THE INVENTION

This invention generally relates to using cardiac signals to monitor patient health, and more specifically to using left atrial pressure signals to anticipate and treat heart conditions.

### BACKGROUND

The human heart has four chambers. A left and right atrium, and a left and right ventricle. The atria receive blood from veins, and the ventricles discharge blood to arteries. The right-side structures receive deoxygenated blood from the body and send it to the lungs to be oxygenated. The left-side structures receive the newly oxygenated blood from the lungs and send it to the rest of the body. The heart similarly contains four major valves: the mitral (bicuspid) valve, the tricuspid valve, the aortic valve, and the pulmonary valve. Artificial heart valves are life-saving medical devices which can be implanted into a heart in order to replicate the functionality of natural heart valves when they fail.

### SUMMARY OF THE INVENTION

Systems and methods for LAP signal processing in accordance with aspects of the invention are illustrated. One aspect includes a method of treating a heart condition of a patient, including: obtaining a left atrial pressure (LAP) signal using an atrial cardiac sensor implanted into a left atrium of a patient, identifying a set of A-waves and a set of V-waves in the LAP signal, calculating an A/V ratio based on a mean pressure amplitude of the set of A-waves and a mean pressure amplitude of the set of V-waves, determining an appropriate treatment for the patient based on the A/V ratio, and providing the appropriate treatment to the patient.

### BRIEF DESCRIPTION OF THE DRAWINGS

FIG. 1 is an atrial implanted cardiac monitoring system in accordance with an aspect of the invention.
FIG. 2 is a block diagram for a cardiac signal processor in accordance with an aspect of the invention.
FIG. 3A is a chart illustrating an example A-Wave and V-wave in accordance with an aspect of the invention.
FIG. 3B is a chart illustrating key points on the example A-Wave and V-wave in accordance with an aspect of the invention.
FIG. 4 is a flow chart illustrating a process for calculating an A/V in accordance with an aspect of the invention.
FIG. 5 is an example left atrial pressure (LAP) signal in accordance with an aspect of the invention.
FIG. 6 is an example spectral analysis of a typical LAP signal in accordance with an aspect of the invention.
FIG. 7A and 7B illustrate a LAP signal reflective of large slow respiration and shallow fast respiration, respectively, in accordance with an aspect of the invention.
FIG. 8 is a de-noised LAP signal in accordance with an aspect of the invention.
FIG. 9 is a parsed LAP signal in accordance with an aspect of the invention.
FIG. 10 illustrates the relationship between an LAP signal and an electrocardiogram (EKG) signal in accordance with an aspect of the invention.
FIG. 11 illustrates a labeled, parsed LAP signal in accordance with an aspect of the invention.
FIG. 12A and FIG. 12B illustrate a baseline V-Wave and an elevated V-wave, respectively, in accordance with an aspect of the invention.

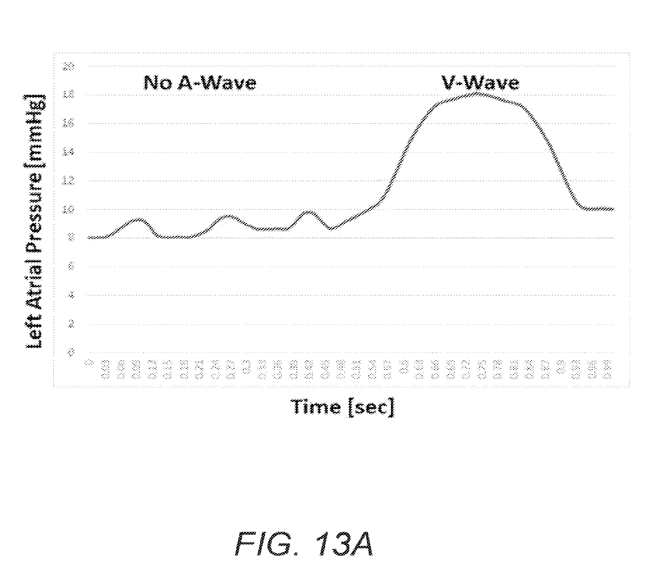

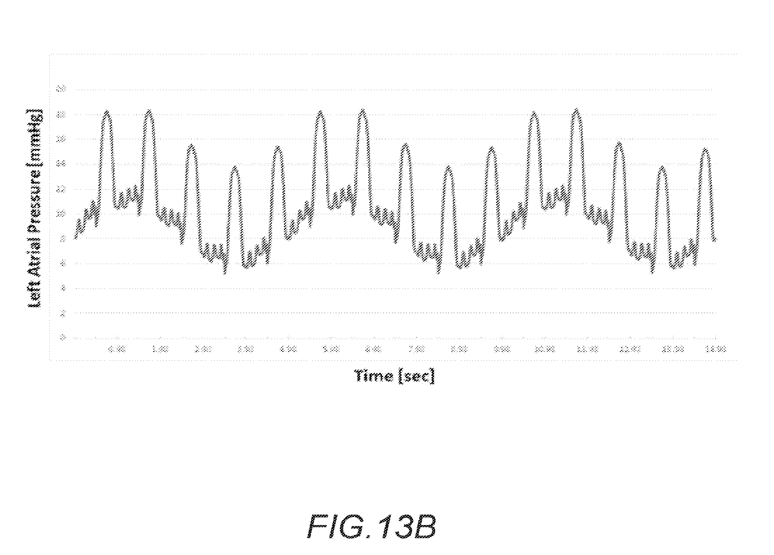

FIG. 13A and 13B illustrates a single A-V cycle, and a longer time-scale LAP signal of a patient in atrial fibrillation, respectively, in accordance with an aspect of the invention.
FIG. 14 is a flow chart illustrating a process for determining cardiac changes over different exertion states in accordance with an aspect of the invention.
FIG. 15 is a chart illustrating change in LAP as a function of change in heart rate in accordance with an aspect of the invention.
FIG. 16 is a chart illustrating change in respiration rate as a function of change in heart rate in accordance with an aspect of the invention.

FIG. 17 is a chart illustrating change in A/V ratio as a function of change in heart rate in accordance with an aspect of the invention.

FIG. 18 is a flow chart illustrating a process for dynamically presenting clinical data based on relevancy to a particular patient in accordance with an aspect of the invention.

FIG. 19 illustrates two main type of structural change during heart failure in accordance with an aspect of the invention.

FIG. 20 illustrates a LAP signal showing an enlarged V-wave pressure resulting from mitral regurgitation in accordance with an aspect of the invention.

FIG. 21 illustrates mitral valve stenosis in accordance with an aspect of the invention.

FIG. 22 illustrates a LAP signal showing an enlarged A-wave pressure resulting from mitral stenosis in accordance with an aspect of the invention.

FIG. 23 is a flow chart illustrating a process for calculating Mitral Valve Index in accordance with an aspect of the invention.

FIG. 24 is a chart illustrating key points on a LAP signal for calculating Mitral Valve Index in accordance with an aspect of the invention.

FIG. 25 is a chart illustrating a minima delta in a LAP signal in accordance with an aspect of the invention.

FIG. 26 is a chart illustrating a large minima delta in a LAP signal in accordance with an aspect of the invention.

FIG. 27 is a chart illustrating a negative minima delta in a LAP signal in accordance with an aspect of the invention.

FIG. 28 is a first process for calculating pulmonary congestion risk index in accordance with an aspect of the invention.

FIGs. 29A and 29B are charts graphically illustrating the first process on an arbitrary sample data set in accordance with an aspect of the invention.

FIG. 30 is a second process for calculating pulmonary congestion risk index in accordance with an aspect of the invention.

FIGs. 31A and 31B are charts graphically illustrating the second process on an arbitrary sample data set in accordance with an aspect of the invention.

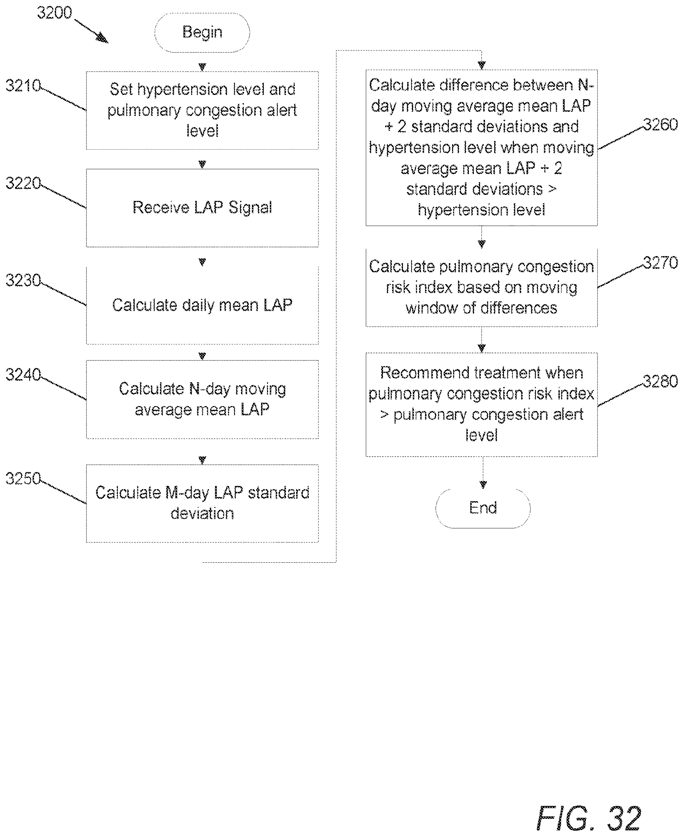

FIG. 32 is a third process for calculating pulmonary congestion risk index in accordance with an aspect of the invention.

FIGs. 33A and 33B are charts graphically illustrating the third process on an arbitrary sample data set in accordance with an aspect of the invention.

DETAILED DESCRIPTION

Despite significant advancements in medical care, cardiovascular disease is a leading cause of death worldwide. Patient outcomes can be significantly more positive when underlying problems are detected earlier.

Cardiac metrics such as heart rate and respiration rate are common measurements that are used in diagnostics and monitoring. In many cases, a patient's disease state can be better understood in the context of the difference in cardiac function between a resting state and an exertion state. However, cardiac function is most often measured when the patient is at rest, e.g. sitting in a doctor's office, laying on an examination table, etc. Measurement of a patient during an exertion state (e.g. moving about, walking quickly, climbing stairs, etc.) can be difficult and/or cumbersome as the monitoring equipment either needs to be able to move with the patient, or a static exertion environment with measurement capabilities must be provided.

Monitoring these metrics continuously (or even at will) at arbitrary times during the day can be difficult without the immediate assistance of a medical professional. While some modern wearable devices (e.g. smart watches) have biometric measurement functions, they can be unreliable in situations where a high degree of accuracy is required. Further, conventional wearable devices are unable to record certain pressure measurements such as (but not limited to) left atrial pressure (LAP) which as discussed herein can be used for cardiac monitoring and treatment.

To address these issues, implantable medical devices with recording capabilities have the potential to enable consistent and frequent measurement of biological functions. With implantable atrial implanted cardiac monitoring systems in accordance with various aspects of the invention, new and more accurate measurements (including LAP measurements) are now obtainable. In order to take advantage of these new data, the systems and methods described herein can record and calculate cardiac metrics which have clinical relevance for both early detection of heart disease as well as other disease states, and treatment direction. In many aspects, the derived cardiac metrics such as (but not limited to) heart rate, respiration rate, mean blood pressure, A/V ratio, etc., are of high accuracy and can be calculated over many different exertion states and/or over a long period of time in order to get an accurate picture of the difference between exertion and rest states. The changes in the ratio between the various cardiac metrics at different states can be used for diagnostics and direction of treatment.

In many aspects, atrial implanted cardiac monitoring systems can measure LAP. LAP is a measurement that has been conventionally estimated as direct measurement (e.g. via transseptal left heart catherization) is difficult and has significant associated risks. LAP can be estimated in a number of ways including during a Swan-Ganz catheterization in which a balloon-flotation technique is used to measure pulmonary capillary wedge pressure. LAP can also be estimated by measuring left ventricular end-diastolic pressure. However, these estimates can be inaccurate and can be insufficient for precision, personalized medicine.

In several aspects, implanted cardiac monitoring systems enable at-will direct recording of LAP at a high sampling rate as a continuous signal. However, merely measuring LAP is insufficient for high quality cardiac monitoring. As discussed herein, LAP signals can be processed to extract clinically significant information regarding a patient on a moment-to-moment basis and be used to determine treatment protocols for medical staff. Of particular note is the A/V ratio described herein has particular clinical relevance. For example, LAP signals on their own may not significantly change during atrial fibrillation (AF), but the derived A/V ratio can indicate the change in disease state. In particular, A/V ratio can be a useful metric when dynamics are informative, as opposed to (or as well as) absolute heart pressure values, such as in the case of measuring change in heart function between a resting state and exertion stateFor example, in many aspects, changes in A/V ratio can be used to identify and subsequently treat any number of conditions including (but not limited to) heart failure (HF), AF, hypertension, pulmonary hypertension, mitral regurgitation, mitral stenosis, aortic stenosis, and cardiac rhythm disorders (e.g. super-ventricular tachycardia, ventricular tachycardia, etc.). As can be readily appreciated, this list of conditions is not exhaustive, and any number of different conditions for which A/V ratio is clinically relevant can be analyzed as appropriate to the requirements of specific applications of aspects of the invention.

Not only are there many different types of cardiovascular disease, there are many different types of patients. As each person is at last somewhat idiosyncratic, patient outcomes can be significantly improved by personalized treatment plans. In order to rapidly generate personalized treatment plans, patients can preliminarily be classified into at least one unique class that attempts to group highly similar patients, and information can be quickly presented to medical professionals based on the classification. Further, depending on the particular classification of a given patient, various cardiac metrics may be analyzed differently. For example, normal ranges may be different for different classifications. By way of further example, certain metrics may be calculated in a different way; A/V ratio may be calculated and/or presented in a different way if a particular patient class is unlikely to show a defined A and/or V wave.

LAP can also be used to calculate heart rate and respiration rate with a high degree of accuracy using systems and methods described herein. Processes for calculation of the A/V ratio is disclosed in detail below. LAP and other cardiac signals and/or metrics in conjunction with patient information can be used by systems and methods described herein to monitor patient health and to produce personalized treatment plans. Cardiac monitoring systems are described below.

Atrial Cardiac Monitoring Systems

At a high level, cardiac monitoring systems include implanted sensors capable of generating LAP signals and processing devices capable of extracting clinically relevant information from the LAP signals. While systems that include implanted sensors are predominantly discussed below, as can be readily appreciated, processes described herein can be performed using LAP signals or estimates thereof recorded using external or temporarily implanted measurement devices.

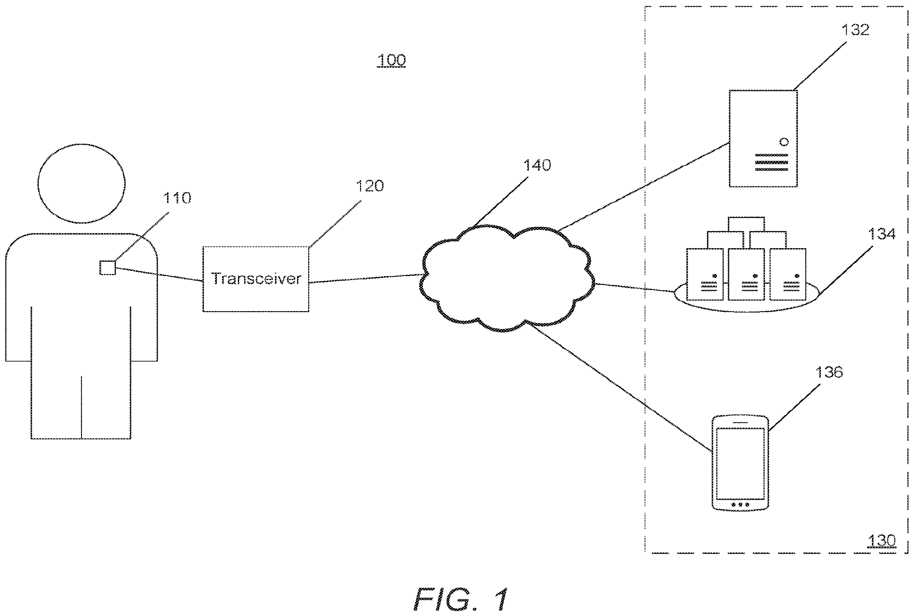

Turning now to FIG. 1, an atrial cardiac monitoring system in accordance with an aspect of the invention is illustrated. System 100 includes an atrial cardiac sensor 110 implanted into the left atrium of a patient. In numerous aspects, the atrial cardiac sensor is a sensor capable of directly measuring heart function and transmitting the recorded data outside of the body In various aspects, the atrial cardiac sensor is capable of recording a LAP signal inside the left atrium. In numerous aspects, the atrial cardiac sensor can also measure other different heart functions including (but not limited to) blood pressure, blood flow rate, heart rate, flow direction, turbulence, and/or any other cardiac metric as appropriate to the requirements of specific applications of aspects of the invention. Records of these metrics over time are referred to herein as "cardiac signals."

In many aspects, atrial cardiac sensors are capable of at least recording multiple cardiac cycles (typically at least 10-20 seconds) with a sampling rate high enough to capture the pressure waveform (typically approximately 100 Hz). In many aspects, the atrial cardiac sensor does not continuously record, and only does so when powered via a wireless power source. As such, data recorded in a single recording session may typically only record 3-25 cardiac cycles In numerous aspects, the recording session lasts for a predetermined amount of time, e.g. between 10-60 seconds. While more cardiac cycles may be recorded via continuous powering, clinically relevant data can be extracted from data describing only this small time window.

The atrial cardiac sensor 110 can transmit recorded cardiac signals to a transceiver 120. In numerous aspects, the transceiver obtains cardiac signals from the atrial cardiac sensor using any of a number of different wireless communication methodologies. In various aspects, the transceiver can control and/or power the operation of the atrial cardiac sensor. For example, the transceiver can use wireless power transfer (e.g. radio-frequency induction) to remotely power the atrial cardiac sensor. In many aspects, powering the atrial cardiac sensor triggers the recording and transmission process. In a number of aspects, the atrial cardiac sensor has an on-board memory which stores recorded data which can later be transmitted to the transceiver. In numerous aspects, the transceiver can direct the atrial cardiac sensor to take a measurement for a predetermined period. In various aspects, the transceiver is capable of directing the atrial cardiac sensor to automatically record a predetermined number of times per day at a predetermined interval. In a variety of aspects, the transceiver further is capable of obtaining an external heart rate measurement via any of the many different types of wearable heart rate monitoring devices, and triggering the atrial cardiac sensor to take a recording in response to an elevated heart rate. Transceivers can include memory used for storing cardiac signal data received from the atrial cardiac sensor for later transmission.

The transceiver can further transmit the data obtained from the atrial cardiac sensor to one or more cardiac signal processors 130. In numerous aspects the data is transmitted via a network such as (but not limited to) the Internet However, any number of different networks, wireless connections, wired connections, or any combination thereof can be used to transmit the data. In various aspects, the transceiver is implemented as a smart phone. In numerous aspects, the transceiver is a wearable device, such as (but not limited to), a smart watch, a chest-worn device, and/or any other wearable as appropriate to the requirements of specific applications of aspects of the invention. As can be readily appreciated, many different devices can be used as transceivers so long as they have appropriate communications and processing capabilities.

Cardiac signal processors are computing devices capable of processing measurements taken by the atrial cardiac sensor and extracting clinically relevant metrics. In numerous aspects, cardiac signal processors are capable of providing personalized treatment plans based on the clinically relevant metrics and patient information. Cardiac signal processors can be implemented any number of different computing platforms such as (but not limited to) personal computers (132), server systems (134), smart phones (136), and/or any other computing platform with sufficient computational power as appropriate to the requirements of specific applications of aspects of the invention. Further, in numerous aspects, the transceiver and cardiac signal processor can be implemented as part of the same hardware platform.

In many aspects, transceivers and/or cardiac signal processors are further capable of identifying when the atrial cardiac sensor needs replacement. In numerous aspects, unexpected changes in received signals can be used to determine the need for replacement. For example, degradation in signal quality, shifts in pressure readings over time that are not substantiated by other measurements, and/or any other change in the signal can be used as appropriate to the requirements of specific applications of aspects of the invention. In a variety of aspects, the expected lifetime of the atrial cardiac sensor can be used as part of the determination. In various aspects, the atrial cardiac sensor can transmit a signal indicating that it needs replacement. In many aspects, atrial cardiac sensors can be incorporated into artificial heart valves, and in turn can indicate when the artificial heart valve needs replacement.

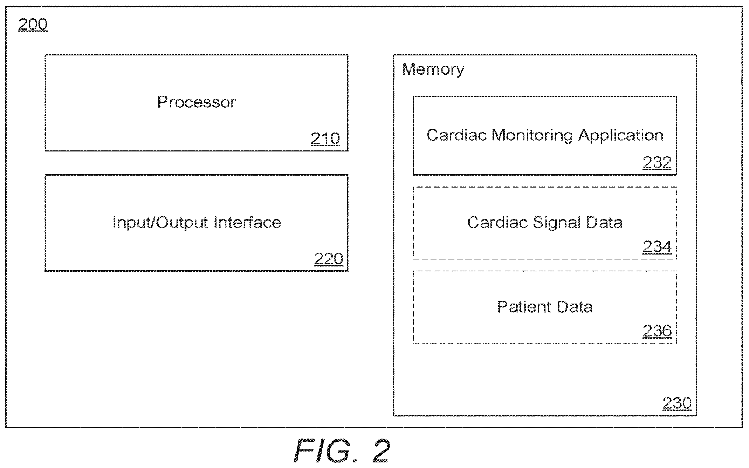

As can be readily appreciated, while a specific architecture for an atrial cardiac monitoring system is illustrated in FIG. 1, any number of different architectures (e.g. those that utilize transceivers and/or cardiac signal processors) can be utilized without departing from the scope or spirit of the invention. Cardiac signal processors are discussed in more detail with respect to FIG. 2 below.

Turning now to FIG. 2, a cardiac signal processor in accordance with an aspect of the invention is illustrated. Cardiac signal processor 200 includes a processor 210. In many aspects, the processor is any logic processing circuit capable of carrying out cardiac monitoring processes described herein. The processor can be implemented using any (or a combination and/or multiple of) central processing units, graphics processing units, field-programmable gate arrays, application-specific integrated circuits, and/or any other logic processing circuit as appropriate to the requirements of specific applications of aspects of the invention. The cardiac signal processor 200 further includes an input/output (I/O) interface 220. The I/O interface can be any circuitry or group of circuits capable of enabling communication with transceivers and/or other computing devices. In various aspects, multiple different communication methods can be implemented using the I/O interface as appropriate to the requirements of specific applications of aspects of the invention.

The cardiac signal processor 200 further includes a memory 230. The memory 230 can be implemented using volatile memory, non-volatile memory, and/or any combination thereof. The memory 230 contains a cardiac monitoring application 232 which is capable of directing the processor to carry out various cardiac monitoring processes such as those described herein In many aspects, the memory 230 may variously contain over time cardiac signal data describing cardiac signals obtained from the atrial cardiac sensor, and patient data describing the patient about which the atrial cardiac sensor data was obtained. As can be readily appreciated, while a specific architecture for a cardiac signal processor is illustrated in FIG. 2, any number of different architectures can be utilized without departing from the scope or spirit of the invention. Cardiac monitoring processes are described in further detail below.

Atrial Cardiac Monitoring and Treatment Processes

Atrial cardiac monitoring and treatment processes in accordance with many aspects of the invention involve the utilization of cardiac signals to produce clinically actionable data. A cardiac signal of particular importance for many applications is the LAP signal, which in turn can be used to produce an A/V ratio with significant clinical relevance. The A/V ratio can be used alone or in conjunction with other cardiac signals and/or cardiac metrics to determine personalized treatment plans for a given patient. For example, in numerous aspects, if A/V ratio drops below 0.6, it can indicate that diuretics should be increased by 50%. By way of further example, if mean pressure increases above 40mmHg without a change in A/V ratio, diuretics should not be increased, and hypertension medication should be increased instead. However, any number of different treatment protocols can be used depending on the condition of the patient as appropriate to the requirements of specific applications of aspects of the invention. Further, the A/V ratio can be used as part of an early warning system for patient health, or for closed-loop/open-loop control of drug delivery. For example, changes in cardiac metrics as discussed herein can be used to automatically trigger appropriate drug delivery, e.g. via an infusion pump, or other automated delivery method. While LAP and the derived A/V ratio are not the only cardiac signals of import, processes for calculating the A/V ratio are discussed first below as a foundation.

A. Calculating A/V Ratio

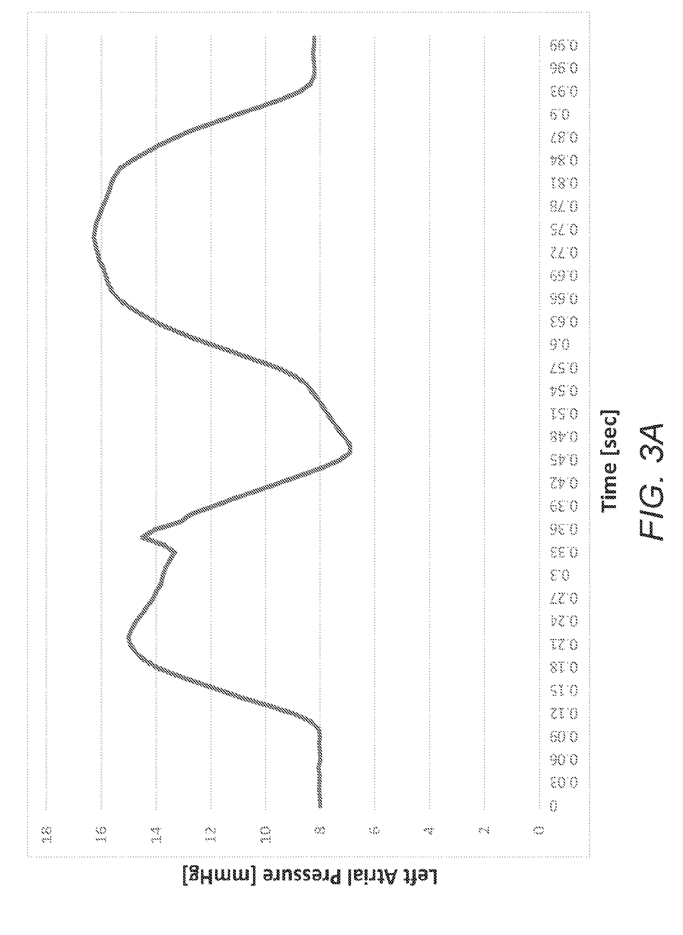

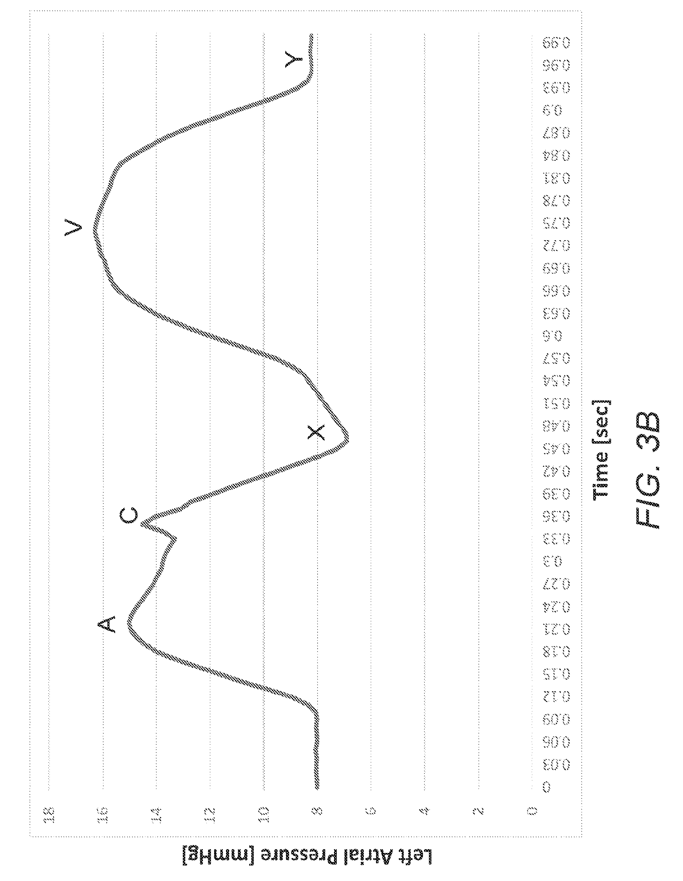

Left atrial pressure over the period of one cardiac cycle can be broken into two major features: the A-wave and the V-wave. The A-wave reflects the pressure surge due to the contraction of the atrium (sometimes referred to as presystolic or atrial systole). The V-Wave reflects the pressure surge due to blood rushing into the atrium after being pushed out of the right ventricle in systole (sometimes referred to as systolic or ventricle systole). Turning to FIG. 3A, an example LAP signal over a single cardiac cycle labeled with the A- and V-wave components in accordance with an aspect of the invention is illustrated. Additionally, five key phases can be mapped onto the LAP signal, which are indicated in accordance with an aspect of the invention in FIG. 3B. The A-wave, as noted above, indicates the right atrial contraction at the end of diastole. The C-wave indicates the mitral valve cusps bulge into the left atrium during early systole. The X descent indicates left atrial relaxation in mid systole. The V-wave again indicates the rapid filling of the right atrium in late systole. Finally, the Y descent indicates the rapid emptying of the left atrium into the left ventricle filling in early diastole.

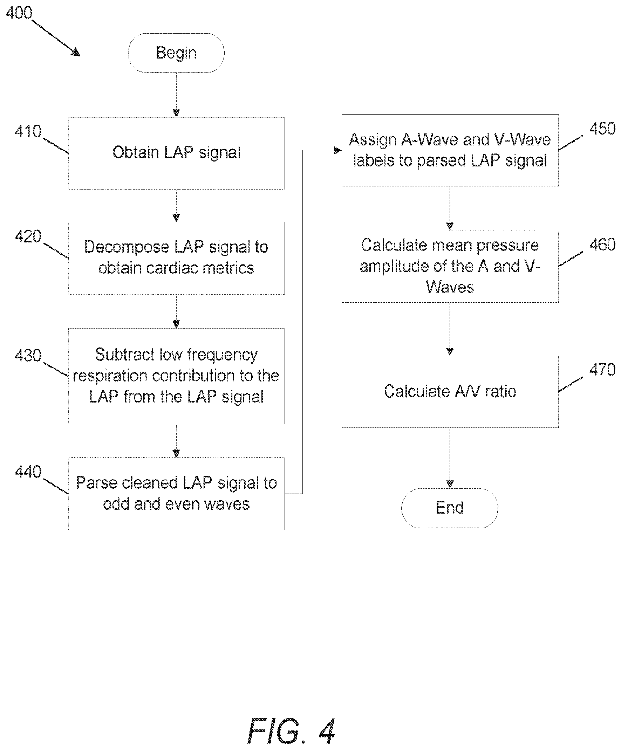

The A/V ratio is the ration between the A-wave mean amplitude and the V-wave mean amplitude. It is a very sensitive parameter because changes in the A/V ratio are earlier and more pronounced than changes in overall mean pressure in response to onset of congestion or edema. While on its face the A/V ratio might appear simple to calculate, due to constant chaos introduced by the living human body, it is in fact a non-trivial task to extract. Turning now to FIG. 4, a process for calculating A/V ratio in accordance with an aspect of the invention is illustrated.

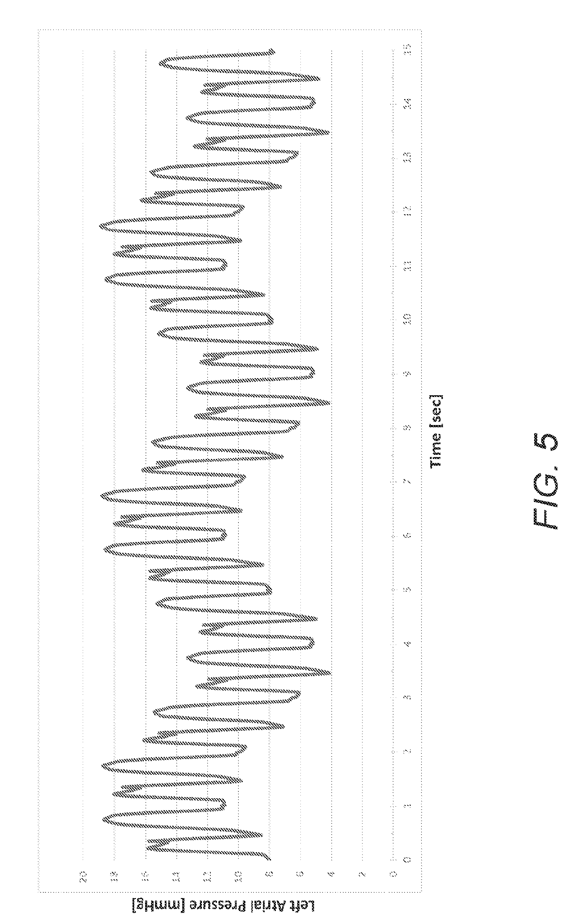

Process 400 includes obtaining (410) an LAP signal. In many aspects, the LAP signal is obtained from an atrial cardiac sensor as described above. While an LAP signal for one cardiac cycle was discussed above with respect to FIGs. 3A and B, an LAP signal over many cardiac cycles is significantly more complicated. In a real-world scenario, it is not always easy to interpret an LAP signal because there can be changes to the different LAP phases. There can also be changes due to pressure generated by respiration that contributes to LAP, typically in a lower frequency band. A real-world example LAP signal in accordance with an aspect of the invention is illustrated in FIG. 5.

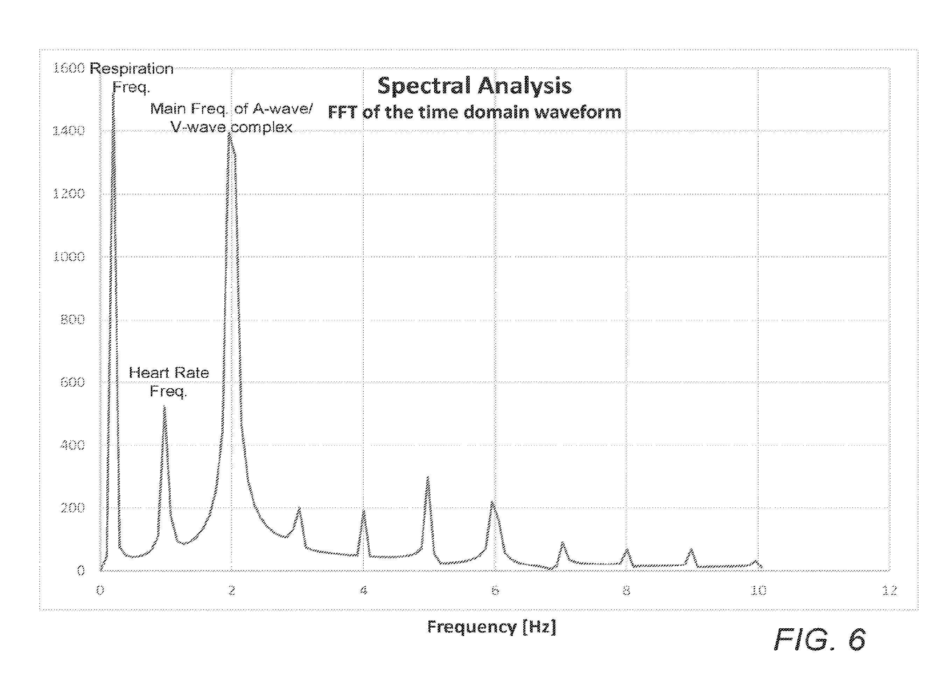

The LAP signal can be decomposed (420) as part of spectral analysis to obtain cardiac metrics. In many aspects, the LAP signal can be decomposed into frequency components using a Fast Fourier Transform (FFT). In various aspects, the LAP signal can be decomposed using wavelet analysis. The decomposed LAP signal provides respiration frequency (typically 0.1-0.5Hz), heart rate frequency (typically 0.8-1.7Hz at rest), and the main frequency of the A-wave/V-wave complex (near 2Hz, approximately double the heart rate). Small peaks at higher frequencies typically are harmonics of the base respiration and cardiac pressure frequencies. An example spectral analysis of a LAP signal in accordance with an aspect of the invention is illustrated in FIG. 6. As can be readily appreciated, other spectral analysis methodologies that can separate respiration rate from heart rate can be used as appropriate to the requirements of specific applications of aspects of the invention. In various aspects, if respiration rate cannot be separated from heart rate, it can indicate that the patient may have been holding their breath.

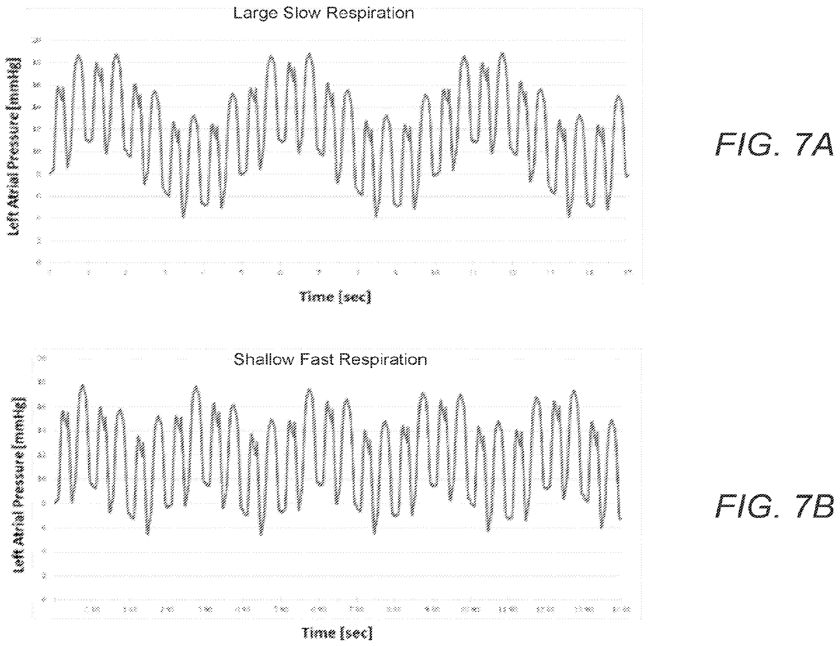

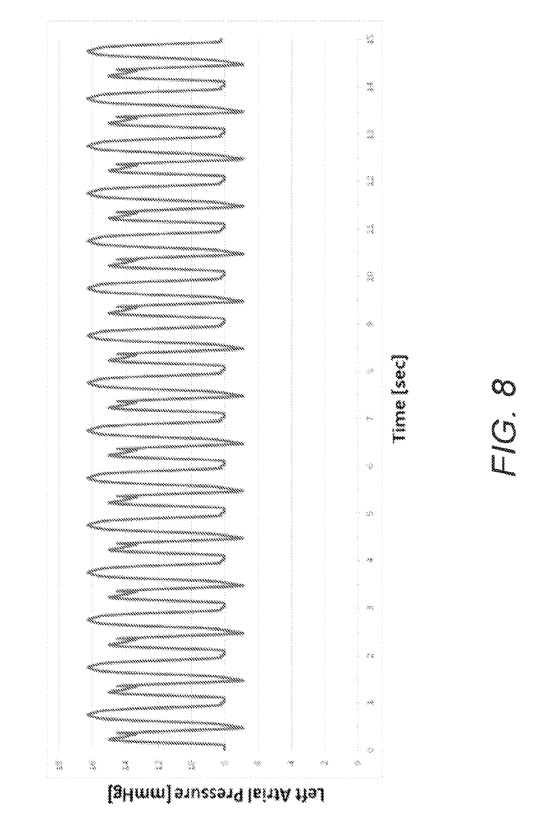

After detecting the respiration and heart rate frequencies, the respiration contribution can is subtracted (430) from the cardiac pressure component of the LAP signal by applying a lowpass filter at a frequency cutoff between the respiration frequency and the heart rate frequency. It is noted that the respiration rate and magnitude can be derived from the signal and can be important in cardiac patients. A shallower faster respiration can indicate shortness of breath which is an important symptom for a range of cardiac conditions such as pulmonary congestion and pulmonary edema. Example LAP signals with respiration components indicating large slow respiration and shallow fast respiration in accordance with an aspect of the invention are illustrated in FIGs. 7A and 7B, respectively. An example cleaned LAP signal with the respiration contribution subtracted in accordance with an aspect of the invention is illustrated in FIG. 8. Typically, heart rate starts around 40-90 beats per minute (BPM) and can go up to 220 BPM during exertion. Typical respiration rate starts at approximately 5-20 respirations per minute (RPM) and can go up to 100 RPM during exertion. However, as can readily be appreciated, these numbers are merely representative of the typical population, and may be different due to idiosyncrasies between individuals.

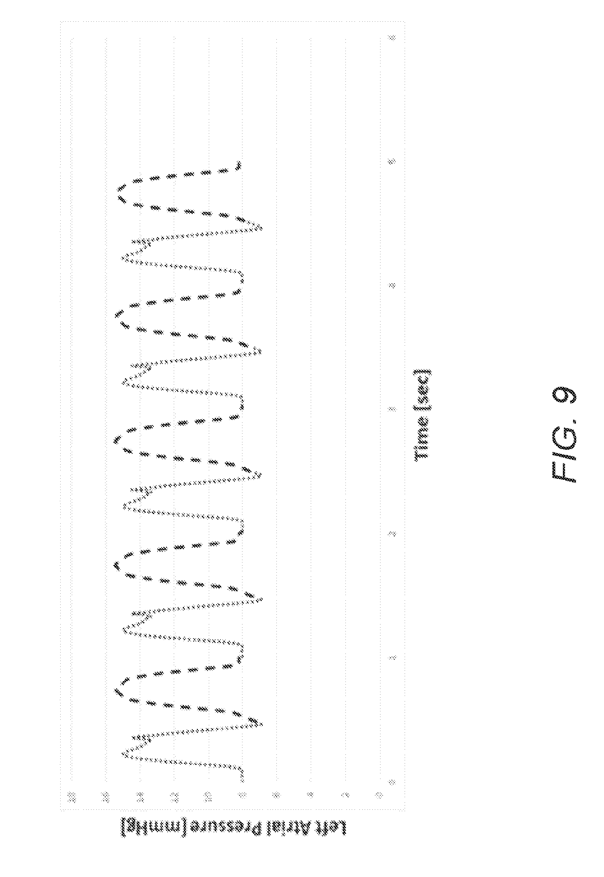

Once cleaned, the LAP signal can be parsed (440) into even and odd waves. In numerous aspects, the waveform is parsed into discrete waves at a frequency around double the heart rate. In many aspects, the local minimum in a short time window centered at double the heart rate is selected as the split point. However, any number of different methodologies can be used to split the waveform. For example, machine learning models can be used as appropriate to the requirements of specific applications of aspects of the invention. A parsed LAP signal in accordance with an aspect of the invention is illustrated in FIG. 9.

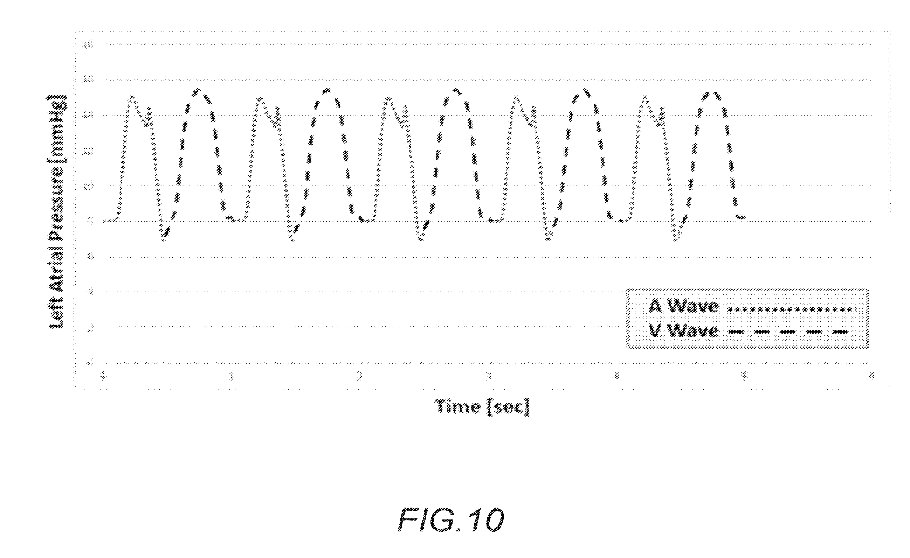

The even and odd waves can then be labeled (450) as A-waves or V-waves. A labeled version of a parsed LAP signal in accordance with an aspect of the invention is illustrated in FIG. 10. Since A and V-waves alternate, all odd waves can be assumed to belong to one group, and all even waves belong to another. A-waves can often be identified as having a double peak (A and C waves), whereas V-waves tend to have a single peak. The X descent is further typically flatter at the bottom than the Y descent, which tends to be pointier. In numerous aspects, a machine learning model can be trained and used to identify A and V-waves within a parsed LAP signal. For example, a machine learning model can be trained using a training data set made of LAP signals that have their A and V-waves annotated. However, other methodologies such as (but not limited to) searching for double peaks within a single wave can be utilized as appropriate to the requirements of specific applications of aspects of the invention. For example, in numerous aspects, for each parsed wave, if the gradient graph of the wave has two zero crossings present aside from the edges, an A wave can be assigned. If this holds true across every other wave, then the labeling can be confirmed. It is important to note that for some patients, the A-wave may not always be present. In various aspects, an inability to detect an A-wave automatically can be flagged to a medical professional for further investigation and/or treatment recommendation.

In various aspects, only a portion of the parsed signal is processed in order to increase computational efficiency. In numerous aspects, the size of the portion can be increased until a confidence threshold is reached. The wave assignments can then be extrapolated across even and odd waves in the entire parsed LAP signal. In a variety of aspects, different portions across the parsed signal can be separately processed to increase trust in the extrapolation.

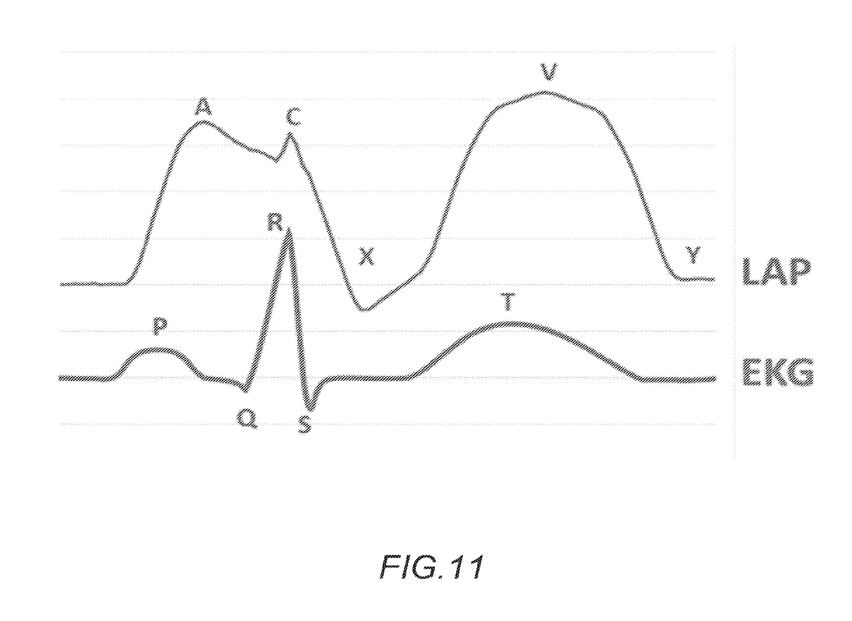

In a variety of aspects, a machine learning model that identifies A and V-waves within the parsed LAP signal can be trained using a training data set containing data collected from real cardiac condition patients together with EKG data. EKG data can be used to determine which wave is which since the A-wave typically occurs before the systole identified in the EKG as the QRS complex, and the V-wave occurs after. This relationship is illustrated in accordance with an aspect of the invention in FIG. 11. The EKG data can be used to rapidly generate sufficiently reliable labeled parsed LAP signal training data without the need for manual labeling of the training data.

Once A and V-waves have been identified, the mean pressure amplitude of the A and V-waves are calculated (460). In numerous aspects, to achieve this, the, the mean pressure and peak-to-peak pressure amplitude of all A-waves and all V-waves are calculated. The A/V ratio can then be calculated (470) as the ration between the A-wave mean amplitude and the V-wave mean amplitude.

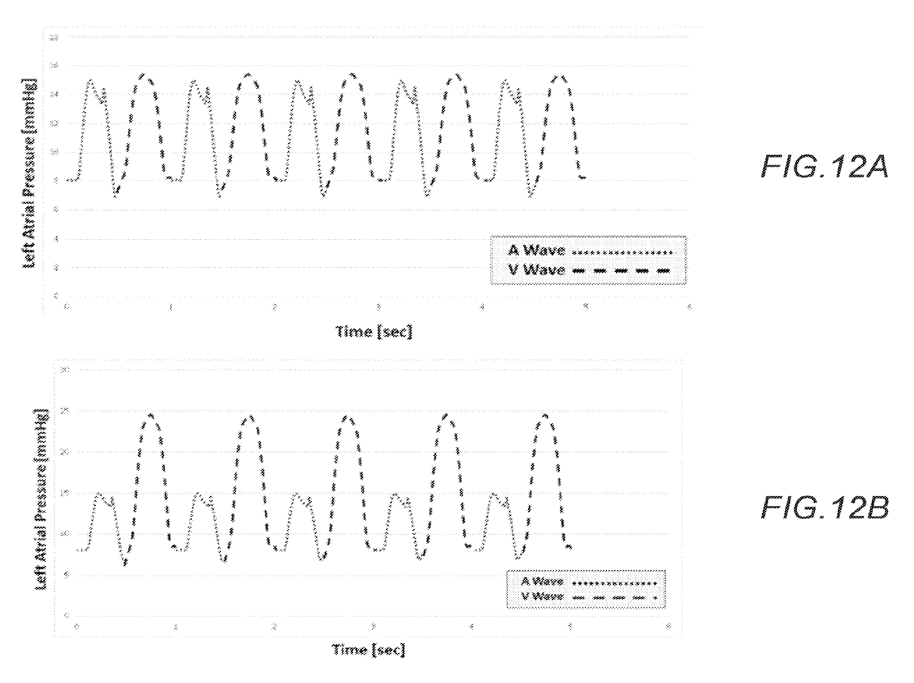

As noted above, the A/V wave is a very sensitive parameter, and therefore proper calculation is often important. To illustrate this, a parsed LAP signal with a baseline V-wave and a parsed LAP signal with an elevated V-wave in accordance with an aspect of the invention are illustrated in FIGs. 12A and B, respectively. In the illustrated example, overall mean pressure is elevated only by 20% from around 12 mmHg to around 14 mmHg, but the A/V ratio drops from around 1 to 0.4, which is a 60% change. A 2 mmHg change in LAP is generally considered to be clinically insignificant, but in this case, it can be identified as a pathological change in the V-wave uncovered by the A/V ratio. Although specific processes are discussed above with respect to the discussion of FIGs. 3-12B, any of a variety of other processes that calculate A/V ratio can be utilized as appropriate to the requirements of specific applications in accordance with various aspects of the invention.

By way of further example, elevated V-wave can be an indication of mitral valve regurgitation (MR). If the mitral valve leaks during systole, blood from the left ventricle flows back into the left atrium at high pressure. The left ventricle can then develop systolic pressures of 100-200 mmHg, which are much greater than the 10-20 mmHg LAP. MR can then be thought of as a primary cause of elevated LAP. Changes in MR can be a critical monitoring factor for treatment of cardiac conditions. For example, worsening of MR over time is an important indicator in the treatment of heart failure. MR can be caused by a number of factors including fluid retention and increased left ventricular preload. The A/V ratio can operate as a more sensitive and more specific parameter to monitor MR than LAP mean pressure, and is therefore useful in the care, monitoring, and treatment of patients with cardiac conditions.

By yet further example, some pathological changes to the A-wave can also be detected as changes in the A/V ration. The most important changes to the A-Wave, especially in heart failure patients, are typically caused by arrhythmia common to heart failure such as atrial fibrillation or atrial flutter. In these conditions the left atrium is fibrillating or fluttering instead of contracting. As such, the left atrium generates no pressure and the A-wave collapses. A collapsed A-wave in a cardiac cycle, and a LAP signal over many cycles showing collapsed A-waves in accordance with an aspect of the invention are illustrated in FIGs. 12A and B, respectively. Again, as can be seen, the overall impact on mean LAP is minimal and often masked by the elevated V-wave, but the A/V ratio is close to zero. In particular, heart failure patients can switch from normal sinus rhythm to atrial fibrillation as they decompensate. Systems that only monitor mean LAP will miss this important clinical indicator which is uncovered via the A/V ratio.

In contrast to a collapsed A-wave, a high A-wave (both alone and in A/V ratio) can suggest low tissue compliance/tamponade, constrictive pericarditis, and/or cardiomyopathy. In many aspects, sharp increases in pressure as reflected in LAP and/or A/V ratio (sudden deflection up or down) can be a critical indicator for patients in high coagulability states, as it can be an indication of pulmonary embolism. As can be readily appreciated from the above examples, the A/V ratio is a clinically significant metric. In many aspects, the A/V ratio can be calculated over time to monitor patient health. Changes in the A/V ratio can be used to uncover specific pathologies that would conventionally go unnoticed for far longer, and direct treatment towards those specific problems. As such, A/V ratio is an important metric to calculate in many scenarios. However, it is not required to calculate A/V ratio in all aspects of the invention, and many other metrics can be calculated and used by cardiac monitoring systems described herein. For example, in numerous aspects, atrial cardiac monitoring systems can monitor patients in multiple sessions over time to create a dynamic health profile. In particular, patients can be instructed to record data at different exertion states to measure cardiovascular performance. Exertion monitoring processes are discussed further below.

B. Exertion Monitoring

Atrial cardiac monitoring systems can record measurements while a patient is ambulatory Typically, blood pressure monitoring via implantable sensors is performed while a patient is at rest, meaning sitting down or laying down. Similar exams from non-implantable devices such as (but not limited to) pulmonary artery catheters are also conducted with the patient laying down on an exam table. Consequently, pressure signals obtained are typically limited to resting conditions. Even if the patient was previously active, recording typically takes place during a resting state. Unfortunately, some changes to pressure only occur when the patient is exerting themselves. Measuring changes during exertion states are therefore important in cardiac condition patient care. As described above, cardiac monitoring systems can include atrial cardiac sensors capable of recording measurements during exertion states, and can process data obtained at different exertion states to extract clinically relevant information.

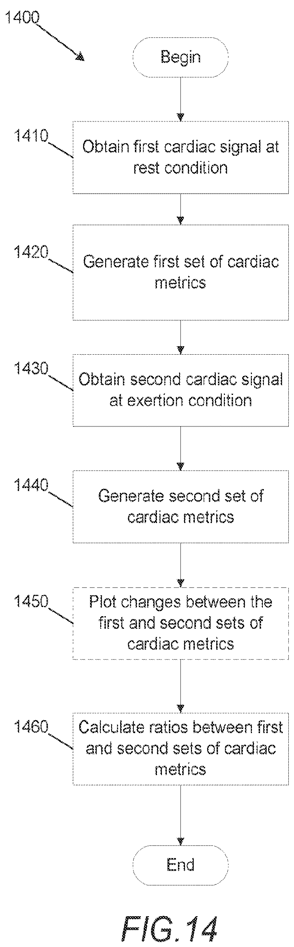

Turning now to FIG. 14, a process for exertion monitoring in accordance with an aspect of the invention is illustrated. Process 1400 includes obtaining (1410) a first cardiac signal recorded while the patient is in a rest condition. In many aspects, the first cardiac signal is a blood pressure signal. In a variety of aspects, the first cardiac signal is a LAP signal. A first set of cardiac metrics are calculated (1420) from the first cardiac signal. In many aspects, the first set of cardiac metrics include heart rate, mean blood pressure, respiration rate, and A/V ratios (if the cardiac signal is a LAP signal). However, depending on the placement (and number of) atrial cardiac sensors implanted, different cardiac signals and cardiac metrics can be obtained and calculated, respectively.

Process 1400 further includes obtaining (1430) a second cardiac signal recorded while the patient is in an exertion state, and calculating (1440) a second set of the cardiac metrics. In many aspects, changes between the first set of cardiac metrics and the second set of cardiac metrics can be plotted (1450) for medical professional review. Further, ratios between the first and second sets of cardiac metrics can be calculated (1460) to derive clinically relevant information.

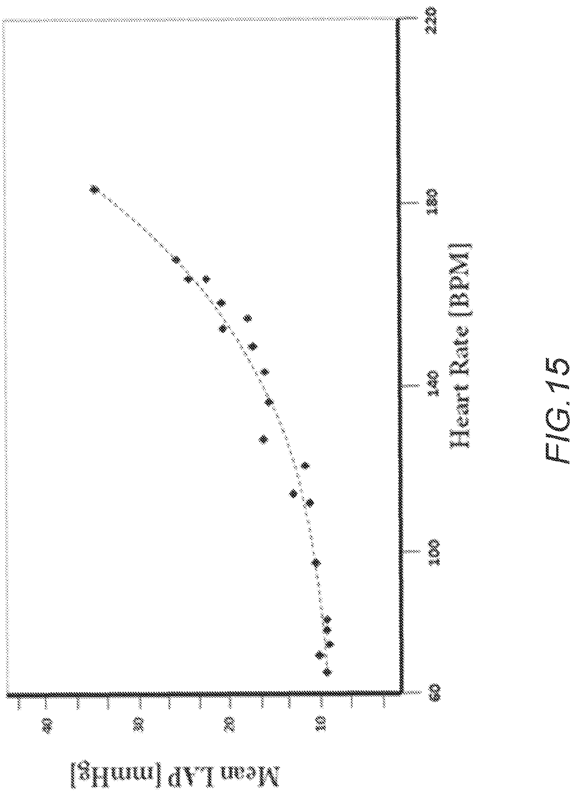

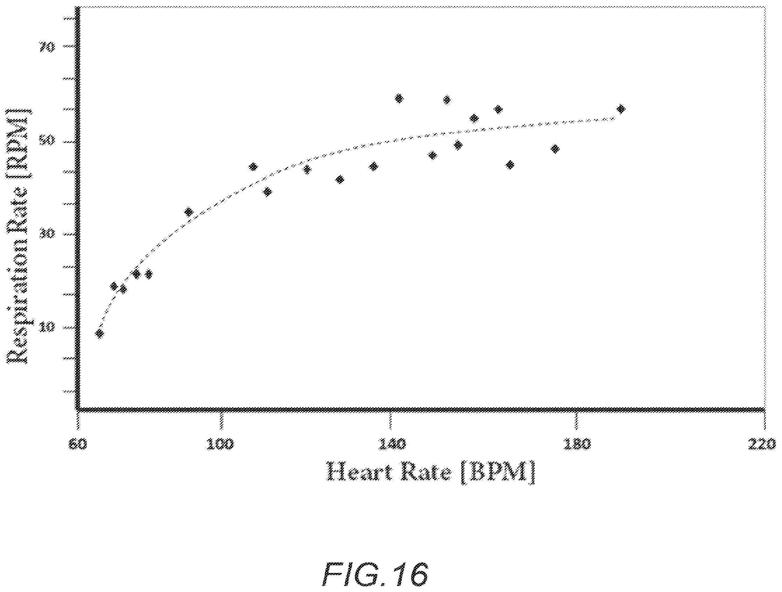

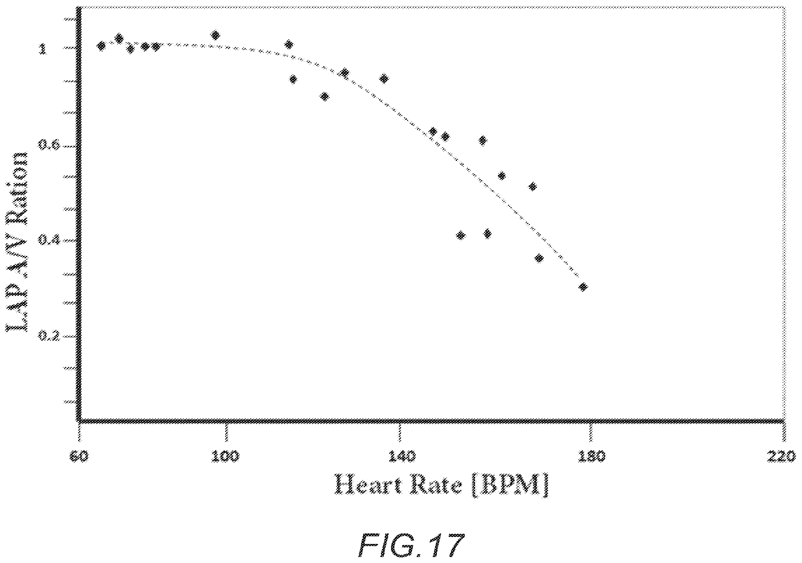

For example, in many aspects, the ratio between mean pressure change and heart rate change (AP/AHR) can be calculated. An example plot of mean LAP as a function of heart rate in accordance with an aspect of the invention is illustrated in FIG. 15. Further, in numerous aspects, the ratio between the respiration rate change and the heart rate change ($\Delta RR/\Delta HR$) is calculated. An example plot of respiration rate as a function of heart rate in accordance with an aspect of the invention is illustrated in FIG. 16. In a variety of aspects, the ratio between mean pressure change and heart rate change (A/V ratio exertion response) is calculated. An example plot of LAP A/V ration as a function of heart rate in accordance with an aspect of the invention is il lustrated in FIG. 17. These metrics (and others) can be used to easily determine whether a patient's response to exertion is worsening, which is a clear indicator of worsening heart condition. This knowledge can be used to immediately begin treatment (or otherwise medically intervene). In numerous aspects, this process can be performed on a regular schedule (or as desired) in order to continuously check cardiac health.

LAP and/or A/V ratio can be used as (or as a component of) a heart failure index which tracks mean pressure variability over time. Tracking of heart condition can provide insight into multiple conditions including (but not limited to) heart failure, coronary artery disease, ischemia, and many others. In various aspects, exertion monitoring can supplement or even replace stress tests that are typically performed using a treadmill or pharmacological induction. As implanted cardiac sensors can collect data at any arbitrary time in a patient's day with minimal impact, tracking can happen at a more granular level than in a clinic. In clinics, typically a test is performed during a single rest state and a single exertion state which are then compared. Performance of these tests take up significant time for patients, and may not accurately reflect their normal state for any number of reasons, including (but not limited to) nervousness due to a medical setting, discrepancies between pharmaceutically induced exertion and exercise-induced exertion, and many others.

In many aspects, these data are collected and plotted on a curve. Heart rate can be used as an indicator for exertion state, and other cardiac metrics such as LAP, A/V ratio, respiration rate, etc. can be plotted over time. Portions of the graph may be colored or otherwise annotated by exertion state for easy readability. In various aspects, the curves for each metric may be labeled with warnings for metrics that are outside of healthy range, constitute unhealthy deltas between resting and exertion states, or any other annotation that maybe clinically helpful. In various aspects, the curves can be used to characterize cardiac condition and/or quantify the severity of heart disease.

While the above discussion focuses on changing heart condition across different exertion states, as can be readily appreciated, similar processes involving measurement over time irrespective of exertion condition can yield valuable information. For example, diurnal changes (night vs day) can provide clinical insight in tracking patient condition. Further, measurement of heart rate over time can enable detection of arrythmias, atrial fibrillation, supraventricular tachycardia, ventricular tachycardia, long QT syndrome, and/or any other heart-rate impacting condition as appropriate to the requirements of specific applications of aspects of the invention. In various aspects, heart rate patterns can be established using one or more methods, including (but not limited to) measuring A/V timing and based on the pressure morphology of the LAP signal. When other measurement tools are available, additional data can be used to confirm the veracity of the measurements. For example, a V-wave typically is expected to coincide with an echocardiogram (ECG) T-wave.

With detailed and constant access to the medical information provided by systems and methods described herein, personalized medicine becomes increasingly feasible. Indeed, systems and methods described herein can utilize data to present information differently to medical professionals based on patient profiles in order to maximize quality of care. Patient classification and personalized treatment processes are discussed further below.

C. Personalized Cardiac Healthcare

Cardiac condition patients are very heterogenous, and similar cardiac signals can mean different things in different patients. As discussed throughout, various systems and methods described herein can obtain a wide variety of cardiac signals at will and generate clinically relevant cardiac metrics that have increased sensitivity for each patient. Systems and methods described herein can be used to classify patients to any of a number of subcategories, and cardiac signal s and metrics can be presented to the medical professionals in a context that best fits the patient's overall classification.

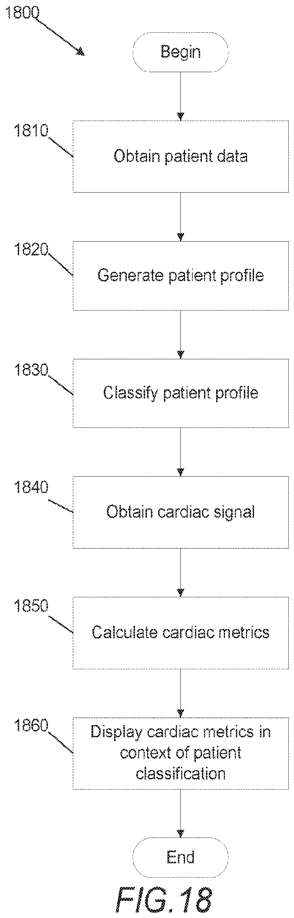

Turning now to FIG. 18, a process for personalized presentation of cardiac information in accordance with an aspect of the invention is illustrated. Process 1800 includes obtaining (1810) patient data. In numerous aspects, patient data includes background patient data. Background patient data can be manually entered by a healthcare professional and/or obtained via electronic health records. The patient can then be parameterized based on their background patient data to generate (1820) a patient profile. Parameters for a patient profile can include (but are not limited to): sex; type of cardiac condition (cardiac condition with preserved ejection fraction (diastolic) - HFpEF; cardiac condition with reduced ejection fraction (systolic) -HFrEF); hypertension (none; mild; severe); pulmonary hypertension group (Group I - primary PH (idiopathic); Group II - secondary PH (driven by elevated LAP); Group III - active lung disease; Other); arrhythmia (none; paroxysmal atrial fibrillation; persistent atrial fibrillation; other); mitral regurgitation (mild; moderate; severe); other valvular pathologies (regurgitation; stenosis); ischemia and/or post-ischemia scar tissue; pacemaker (yes; no); and/or any other relevant parameters as appropriate to the requirements of specific applications of aspects of the invention.

Patients are classified (1830) based on the parameterized patient profile. In numerous aspects, each unique combination of parameter values can be treated as a separate class. In numerous aspects, the patient profile is provided to a machine learning model which produces a classification of the input profile. Classification using machine learning is discussed in further detail below. Patients in each class present more homogenously, and it can be assumed that the LAP waveform for each patient in that class can be interpreted in the same or a similar way. One or more cardiac signals are then obtained (1840) and cardiac metrics are calculated (1850). In numerous aspects, depending on the classification of the patient from which the cardiac signal was recorded, the way cardiac metrics are calculated can differ to be more appropriate to the type of heart disease the patient presents with. The cardiac metrics can then be displayed (1860) to medical professionals in context of the classification via a user interface. In numerous aspects, the classification is presented with the metrics. In various aspects, different ranges for normal/abnormal may be presented for one or more cardiac metrics based on the classification. In a variety of aspects, different cardiac metrics may be highlighted as more likely to be clinically relevant based on the classification. In many aspects, different treatment methods may be suggested based on the cardiac metrics and the classification. As can be readily appreciated, any number of different personalized medicine applications can be implemented without departing from the scope or spirit of the invention. Turning now to classification using machine learning, several viable alternatives are presented. In many aspects, a training data set which includes patient profiles annotated with classifications is generated and used to train a supervised machine learning model. In various aspects, the classifications are assigned respective normal cardiac metric ranges. Provisioning of a patient profile to the trained machine learning model can produce a classification of said patient profile, which in turn can be used to interpret the cardiac metrics.

In numerous aspects, the supervised machine learning model which is trained is a random forest model, or a support vector machine. In the case of the random forest model, multiple decision trees can be generated to produce a final model prediction. In contrast, the support vector machine can maximize the boundary between different classes that maximizes the margin between them. In various aspects, more than one model is used. When more than one model is used to classify a patient profile, both classifications may be provided. In some aspects, a definitive classification is only provided if the outputs of the models agree. If there is disagreement, this can be flagged to the medical professional. Further, classification confidence values can be provided signifying the classification probability produced by the model. If the confidence value is below a predetermined threshold (e.g. between 75-99%, although this number can be tuned to the risk comfort of the user), then no positive classification may be provided.

As can readily be appreciated, other supervised machine learning models can be used to produce classifications without departing from the scope or spirit of the invention. In various aspects, an unsupervised machine learning model is used in order to eliminate the burden of generating training data. While in some cases accuracy may be lower, unsupervised learning enables an unbiased classification process which may reveal new insights. For example, a clustering approach can be used on agglomerated patient data into which the instant patient profile is introduced.

In many aspects, when machine learning is utilized for classification, the patient's LAP signal and/or derived cardiac metrics may be incorporated into the patient profile and provided to the machine learning model in order to further assist with the classification process. In various aspects, dimensionality reduction may be applied based on waveform features prior to provisioning the machine learning model.

Utilization of machine learning for classification can be particularly useful when integrated into an electronic health record (EHR) system. The EHR may already consist of patient profiles annotated with patient outcomes and diagnoses. In this way, the EHR itself can be used as a training data set, or as the agglomerated set of patient profiles in the case of unsupervised learning. With a sufficiently well-populated EHR, classification can rise to the level of disease cluster or even individual disease level classification, over even healthy cardiac metric ranges. In some aspects, healthy cardiac metric ranges can be further derived in the context of an EHR by observing the range of cardiac metrics at discharge across the EHR population for that particular class.

D. Implant Monitoring

Overall cardiac health monitoring remains is important function. For many patients who are fitted with an atrial cardiac sensor, additional implants may be (or already be) implanted to maintain cardiac health. However, implanted cardiovascular medical devices may deteriorate over time or be damaged or otherwise impaired via accident. LAP and/or A/V ratio can be used to measure the functionality of implantable medical devices such as shunts and/or heart valves. In numerous aspects, the pressure difference between different sides of a shunt or valve can be used to build a pressure profile, which in turn is suggestive of blood flow. Measurements can be taken using a sensor implanted on either side of the shunt/valve, or via a sensor on one side of the shunt/valve and an external measurement modality. In some aspects, valves and/or shunts may include cardiac sensors capable of pressure measurements as described herein on one or both sides capable of making the requirement measurements.

For example, mitral valve diseases are common forms of cardiovascular disease. There are two main mitral valve abnormalities: mitral regurgitation and mitral stenosis. Mitral valve diseases are often contributing factors of heart failure. In many aspects, mitral valve disease state can be monitored through the use of a Mitral Valve Index (MVI) which reflects the degradation of the mitral valve. MVI can be calculated based on LAP measurements over time, and updated as new LAP measurements are taken. When the MVI exceeds a prespecified level, systems and methods described herein can be used to alert that a mitral valve intervention is needed (e.g. surgical percutaneous valve repair). In numerous aspects, artificial heart valves are monitored using MVI. If the artificial heart valve begins to degrade, MVI can similarly be affected and used to trigger an alert and subsequent repair or replacement.

As can be readily appreciated, while sensors predominantly configured to be implanted in an atrium are discussed above, similar sensors can be made which measure pressure in other regions of the circulatory system, and therefore such pressure differentials can be obtained outside of the left atrium. In many aspects, a mitral valve index is calculated in order to monitor implant status and/or overall cardiac health.

Mitral valve disease can lead to changes in pressure and flow in the left atrium. As noted above, there are two main mitral valve abnormalities: mitral regurgitation and mitral stenosis. In mitral regurgitation, the valve fails to close properly and there is a reverse leak of blood from the left atrium backwards into the left ventricle. Since the ventricle develops much higher pressure than the atrium, the regurgitation causes a pressure spike in the atrium with timing corresponding to the ventricular systole. In numerous aspects, on a LAP signal this appears as an elevated V-wave. Mitral regurgitation is common in heart failure patients.

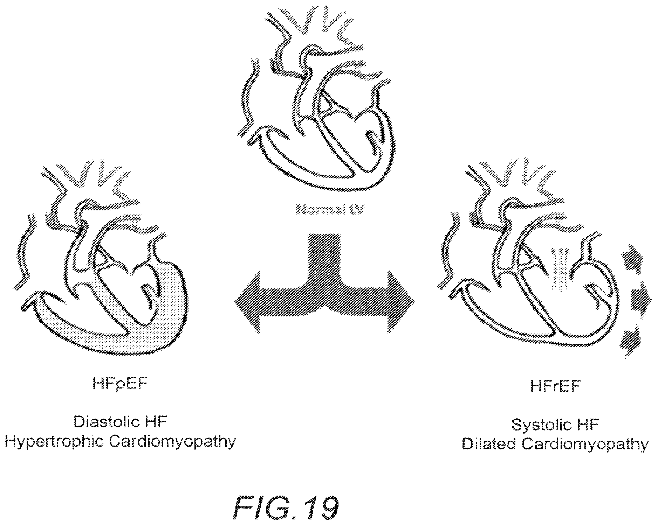

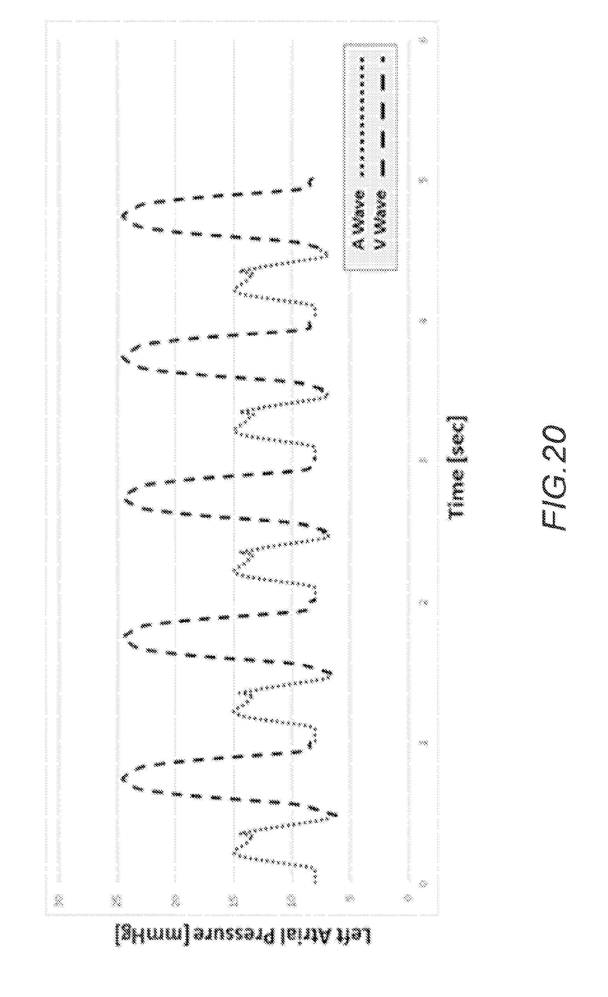

The two main types of heart failure that are associated with two different structural changes in the heart: Heart Failure with reduced Ejection Fraction (HFrEF) and Heart Failure with preserved Ejection Fraction (HFpEF). About 65% of HF patients are HFrEF patients. They have an enlarged heart and reduced Ejection Fraction. If the heart is severely enlarged the mitral valve leaflets are pulled away from each other and fail to close. The remaining 35% of HF patients are HFpEF patients. The heart ventricle muscle walls are thicker than normal, fibrotic, and stiff. The mitral valves typically do not leak in HFpEF, but the stiff ventricle creates a diastolic disfunction in which the ventricle filling by the atrium is longer and generates higher pressure in the atrium. A graphical depiction of the different structural changes in HFrEF and HFpEF hearts compared to normal is illustrated in FIG. 19. Systems and methods described herein can parse LAP signal waveforms to diagnose both HFrEF and HFpEF, and tell the difference between them. In HFrEF mitral valve regurgitation tends to be more common, and presents as a high V- wave (e.g. a peak V-wave LAP of over 25 mmHg) even in moderate mean LAP values (5 - 25 mmHg). In HFpEF, both the A-Wave and the V-Wave tend to be elevated, and therefore the mean LAP is more elevated (e.g. above 20 mmHg) while the V-Wave is also moderately elevated (e.g. also around 20 mmHg). By way of example, a LAP signal showing enlarged V-wave pressure resulting from mitral regurgitation in accordance with an aspect of the invention is illustrated in FIG. 20. It is important to note that aortic stenosis can also cause elevated A-wave pressure.

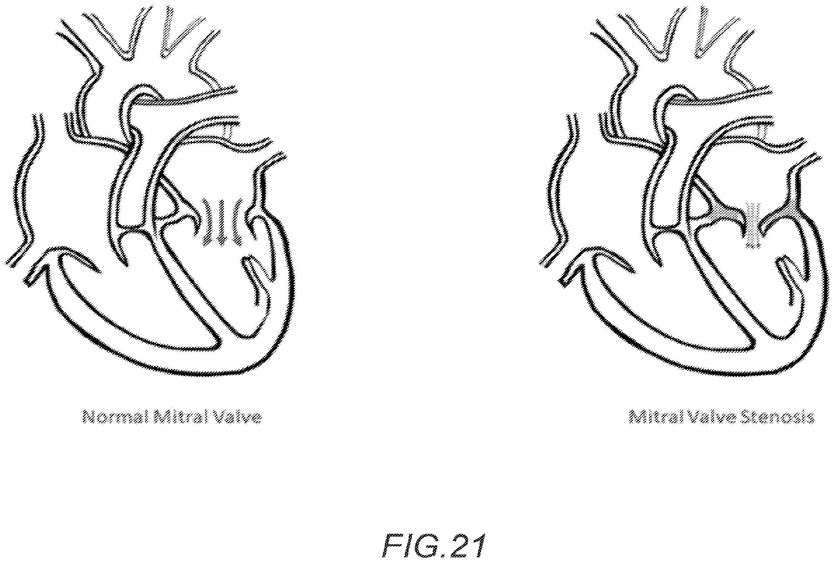

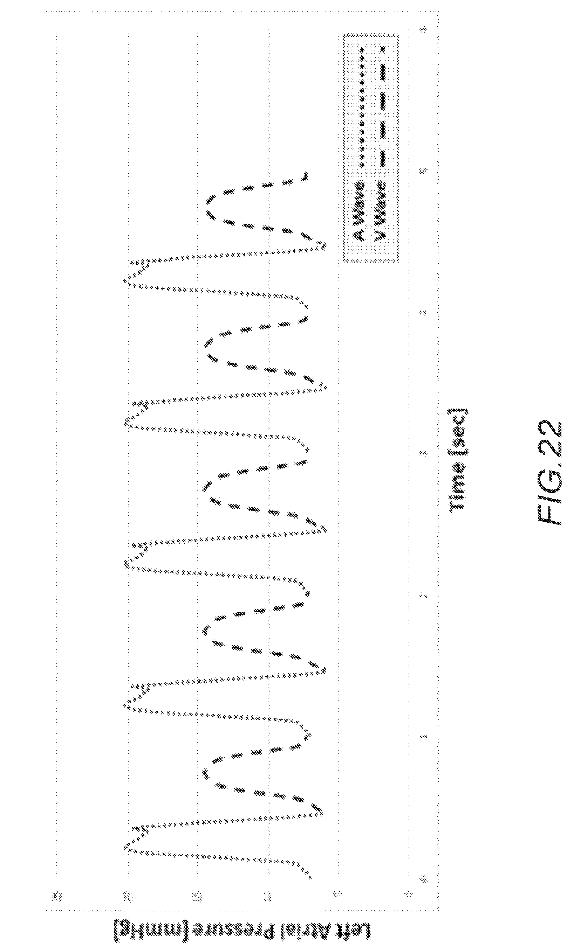

Mitral stenosis can occur when the valve is diseased, calcified, or otherwise enlarged with thicker leaflets, and fails to fully open. During atrial contraction, the mitral valve normally is open wide to allow blood to flow into the ventricle. A stenosed valve opens only slightly, and therefore the pressure in the atrium is elevated. A graphic depiction of a normal mitral valve compared to mitral valve stenosis is illustrated in FIG. 21. In a LAP signal, this is reflected by an elevated A-wave. An example LAP signal with an elevated A-wave due to mitral valve stenosis in accordance with an aspect of the invention is illustrated in FIG. 22.

In order to detect and monitor mitral regurgitation and/or mitral stenosis, cardiac sensors as described herein can be used to record LAP signals. Multiple LAP signals can be taken over time and be analyzed to determine the health of a patient's mitral valve. In numerous aspects, cardiac metrics can be used to quantify the severity of the mitral valve state. In many aspects, a Mitral Valve Index (MVI) is calculated using A/V ratio and the minimum pressure point between the A-wave and the V-wave. When the MVI reaches above a threshold value, an indicator can be provided that intervention is necessary. In many aspects, the intervention is a surgical percutaneous valve repair. In some aspects, the intervention is a surgical percutaneous valve replacement. In various aspects, the intervention is the replacement of an artificial heart valve.

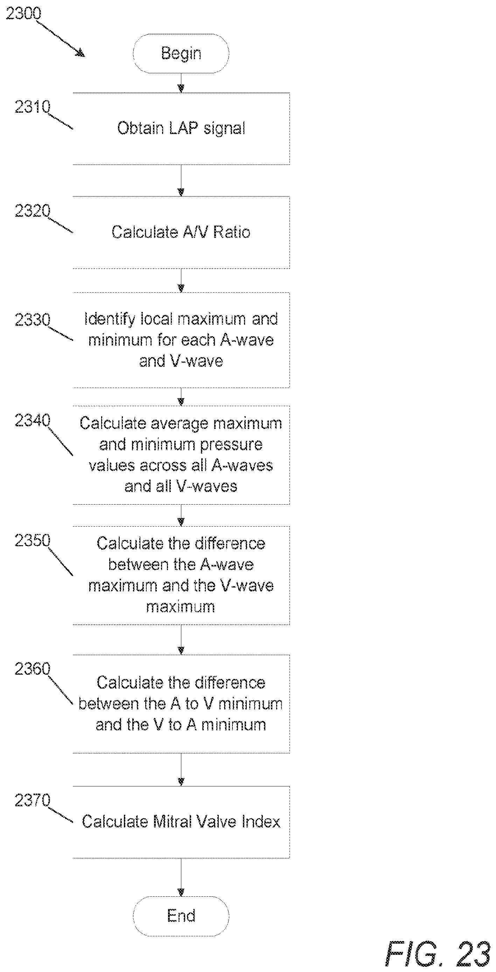

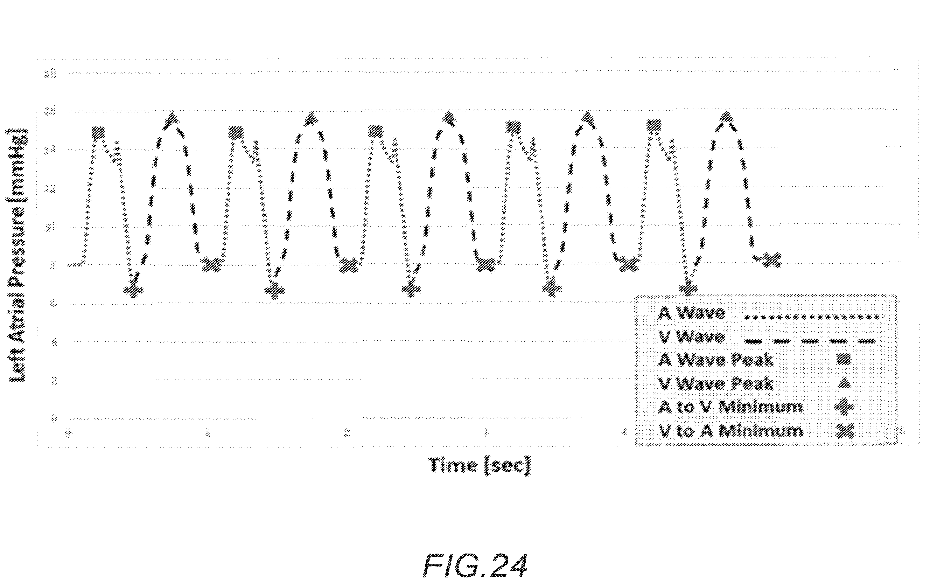

Turning now to FIG. 23, a process for calculating MVI in accordance with an aspect of the invention is illustrated. Process 2300 includes obtaining (2310) a LAP signal. In numerous aspects, multiple LAP signals are obtained. From the LAP signal(s), A/V ratio is calculated (2320). During the A/V ratio calculation, A-waves and V-waves are identified. For each A-wave and V-wave, a local maximum and minimum are identified (2330). In numerous aspects, the minima occur at the A to V, and V to A transitions. The minima at the A to V wave transition is sometimes referred to as the "X depression". Similarly, the minima at the V to A wave transition is sometimes referred to as the Y depression. In various aspects, the maxima occur at the peaks of the respective wave. An example normal LAP signal marked with minima and maxima in accordance with an aspect of the invention is illustrated in FIG. 24. Typically, the A to V transition minimum is slightly higher than the V to A transition minimum.

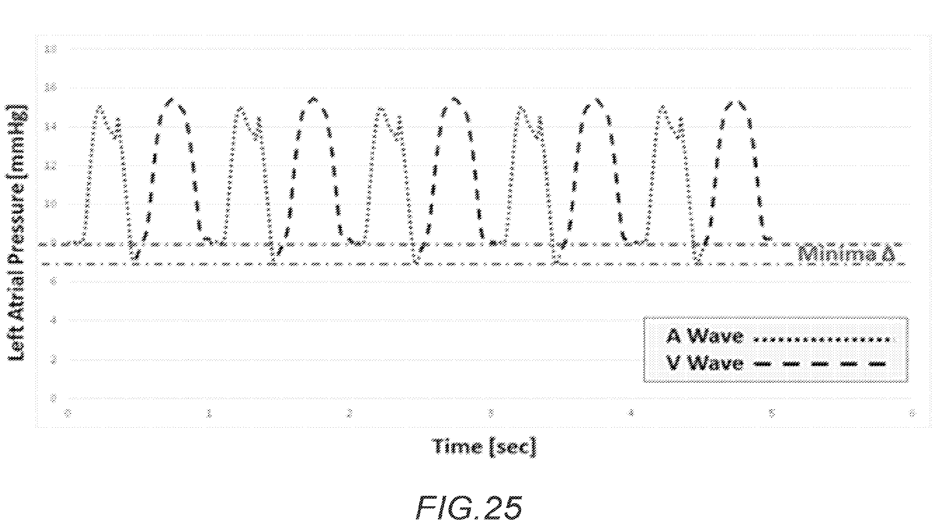

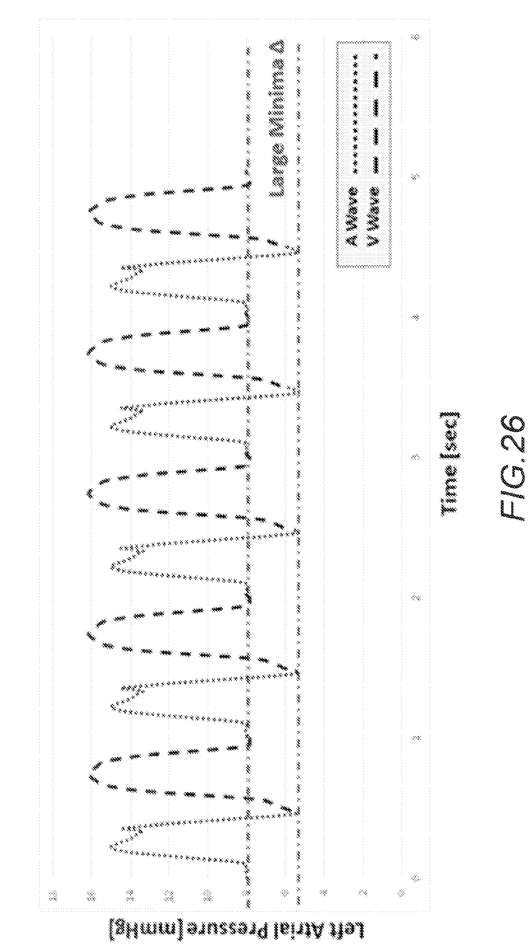

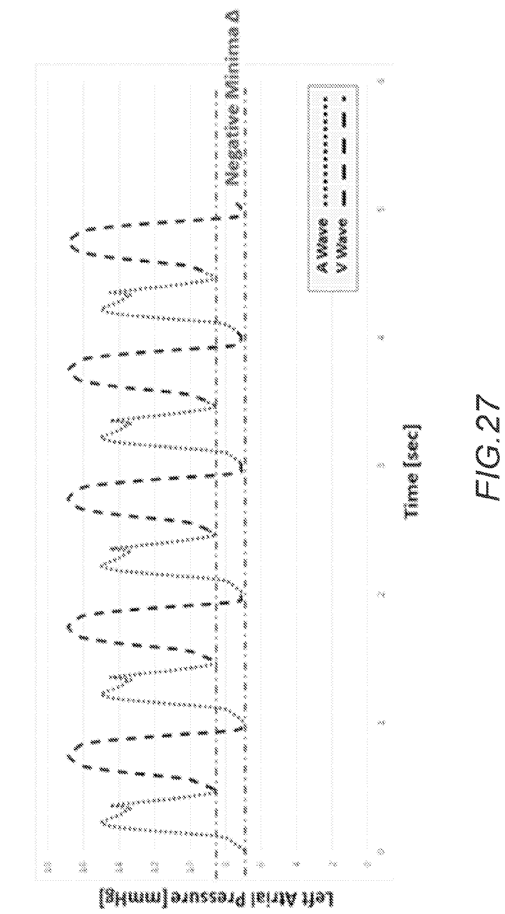

The average maximum pressure and average minimum pressures are calculated (2340). The difference between the average A-wave maximum and the average V-wave maximum is calculated (2350), ΔMax. The difference between the average A to V minimum and V to A minimum is calculated (2360), ΔMin. In numerous aspects, an increased ΔMin can indicate a diastolic disfunction. An example normal ΔMin in accordance with an aspect of the invention is illustrated in FIG. 25. In contrast, an example increased ΔMin in accordance with an aspect of the invention is illustrated in FIG. 26. In various aspects, an inverted ΔMin (e.g. a negative ΔMin resulting from a greater average V to A pressure compared to average A to V pressure) can indicate a late contraction of the ventricle in conditions such as heart block or long QT syndrome. An example negative ΔMin in accordance with an aspect of the invention is illustrated in FIG. 27.

The A/V ratio, ΔMax, and ΔMin are then factored together to calculate (2370) the MVI. In many aspects, MVI can be calculated as:

$$MVI = (A/V\,Ratio)\left(\frac{C1}{\Delta Max}\right)\left(\frac{C2}{\Delta Min}\right)$$

where C1 is the ΔMax of a healthy heart, and C2 is the ΔMin of a healthy heart. Typically, C1 and C2 are between 1 and 2, however, C1 and C2 values can often range between 1 and 5. In numerous aspects, the MVI is used to determine an appropriate time for surgical intervention. In many aspects, an MVI > 1 is considered normal, whereas an MVI < 1 can suggest a need for intervention. In various aspects, MVI is calculated using LAP signals recorded during a patient rest state, and calculated again using LAP signals recorded during a patient exertion state. In some aspects, MVI calculations involve using both A/V ratio and ΔMax/ΔMin values calculated during both exertion and rest states. In a variety of aspects, respiration rate and respiration pressure magnitudes (which can be derived from LAP as discussed above) are also used in conjunction with MVI to monitor cardiopulmonary states of a patient. Changes in lung effect pressure can be monitored and used to track and treat lung conditions. As can be readily appreciated, while the above is discussed with specific reference to LAP, in many aspects, cardiac sensors can equally be implanted in the right atrium and the right atrial pressure (RAP) can be analyzed to detect and monitor tricuspid valve abnormalities.

E. Cardiopulmonary Monitoring

heart failure (HF) is a condition where a patient's heart is impaired and cannot generate enough blood output to supply the demand of the patient's organs for oxygen. When this condition becomes chronic, permanent changes appear in the heart structure and hemodynamics which often result in chronic symptoms that impair quality of life. The most common symptom is pulmonary congestion, leading to HF often being referred to as congestive heart failure (CHF).

Pulmonary congestion is a result of pulmonary hypertension and poor blood flow through the lungs. Heart failure elevates the blood pressure in the heart left atrium which drains blood from the lungs. When left atrial pressure (LAP) is elevated, the blood pressure in the lungs is also elevated, and cause fluid to defuse from the blood capillaries in the lungs to the air cavities (alveoli) in the lungs, causing congestion. In severe pulmonary congestion, the patient is effectively drowning and cannot get enough air into their lungs. This often requires hospitalization to treat.

Treatments include medication to try and minimize pulmonary congestion. Medications include diuretics to reduce general fluid volume of the patient and anti-hypertension drugs to lower blood pressure. High dosages tend to negatively impact quality of life by causing frequent urination and hypotension related fatigue. Accordingly, optimizing medical management is key to maintaining quality of life for HF patients.

Conventionally, patients monitor their heart failure by monitoring blood pressure, heart rate, pulse, weight, and how they feel (e.g., energy level, clothes feeling tight, swelling, shortness of breath, coughing, etc.). These are often subjective and can be difficult for patients to express and doctors to understand. Further, as noted above, conventional wearable devices are unable to record certain pressure measurements such as (but not limited to) left atrial pressure (LAP) which as discussed herein can be used for cardiopulmonary monitoring and treatment. Patient outcomes can be significantly more positive if worsening of pulmonary congestion could be predicted so it could be more aggressively treated, for example by increasing drug dosage before congestion gets too severe that it requires hospitalization.

In many embodiments, a pulmonary congestion risk index is calculated as the area under the mean LAP signal curve above a hypertension level (sometimes also referred to as a "LAP threshold"), in order to monitor a patient. In numerous aspects, at least one LAP signal is recorded daily. Each day, the new LAP signal is analyzed in conjunction with previously recorded daily LAP signals for the patient to trend the data and determine the risk of developing pulmonary congestion. The risk can be represented by a pulmonary congestion risk index. Further, due to noise in the LAP signals and/or the temporal choppiness of their acquisition, filtering and/or averaging techniques can be applied. When the pulmonary congestion risk index exceeds a threshold value, an alert can be provided which can trigger treatment of the patient to prevent severe congestion.

Congestion is caused by diffusion of fluid from the blood to the lung cavities. The speed of diffusion is controlled by the pressure gradient from the blood to the air cavities. If LAP is elevated then pulmonary blood pressure is also elevated, and fluid buildup in the lungs is faster. Excess fluid is usually slowly evacuated from the lungs by reabsorption and exhalation. It the fluid buildup rate is higher than the fluid evacuation rate, fluid builds up in the lungs and can lead to severe congestion. Therefore, integrating elevated pressure over time can be a predictor of pulmonary congestion.

Pulmonary congestion risk indices are a measure of the severity and length of time for which a patient's mean LAP exceeds a hypertension level. In numerous aspects, the hypertension level is 20 mmHg which can be considered as high pressure in the left atrium. But chronic HF patients who are medicated can sustain much higher pressures and their physician may choose a higher threshold. Similarly, some patients may benefit from a lower threshold which reflects high pressure for their heart depending on their particular condition. An index time window can be predetermined for the analysis, typically between three and thirty days. The pulmonary congestion risk index can be calculated by finding all instances in the time window in which the daily mean LAP is above the threshold and summing the differences between the mean LAP and the threshold for all instances in the time window.

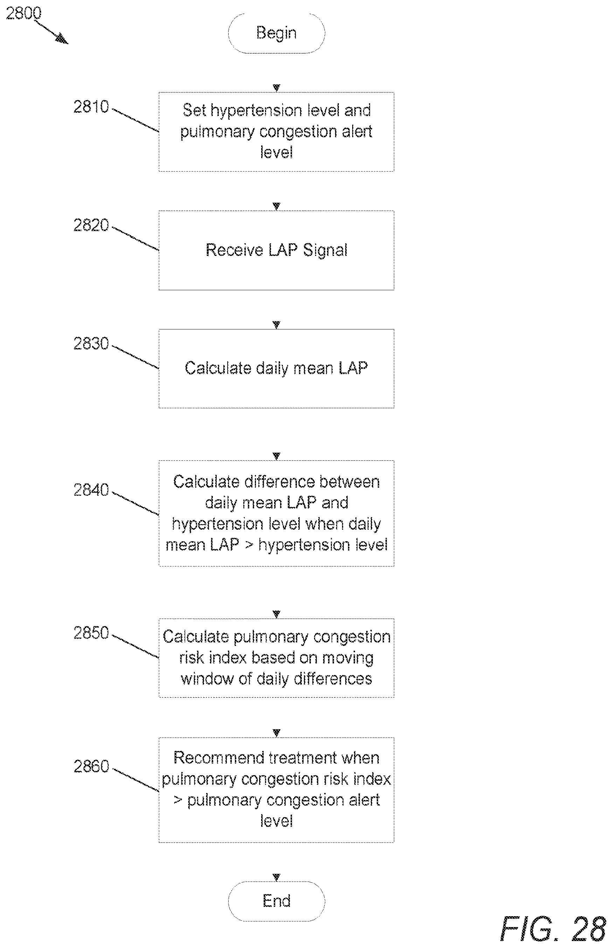

Turning now to FIG. 28, a first process for calculating pulmonary congestion risk index in accordance with an aspect of the invention is illustrated. Process 2800 includes setting (2810) the hypertension level and pulmonary congestion alert level. In numerous aspects, when the hypertension level is 20mmHg, the alert level is set at 30mmHg. The alert level can be set to any arbitrary value depending on the specific patient. In numerous aspects, the hypertension level is 20mmHg, although this number can be varied depending on the particular patient as discussed above. A LAP signal is received (2820) on a daily basis, and the daily mean LAP is calculated

(2830) for each day. In many aspects, the LAP signal is recorded by an atrial cardiac sensor. The LAP signal can be received at a cardiac signal processor via a transceiver as discussed above. In numerous aspects, multiple LAP signals are recorded every day and are averaged together In numerous aspects, LAP signals are recorded daily during a similar exertion state, e.g., during rest.

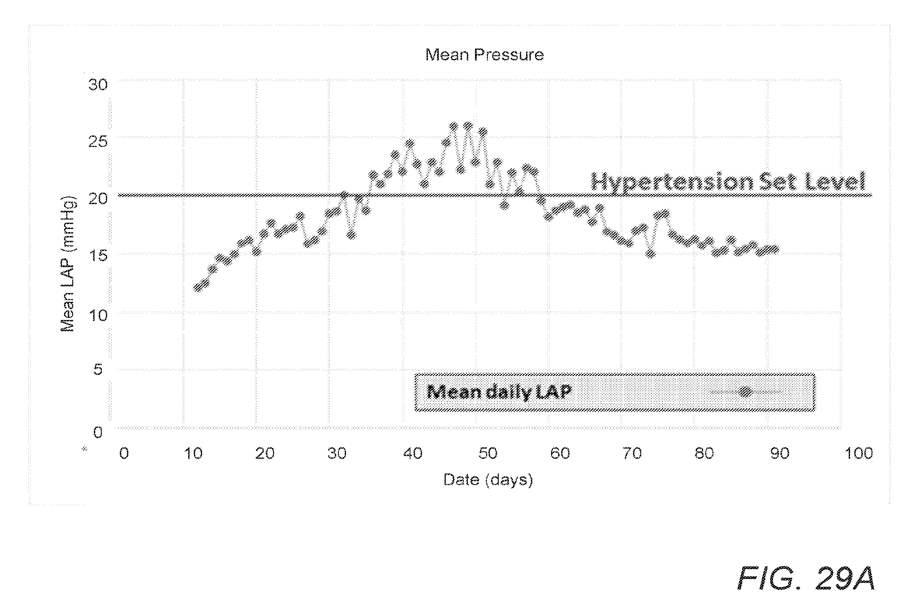

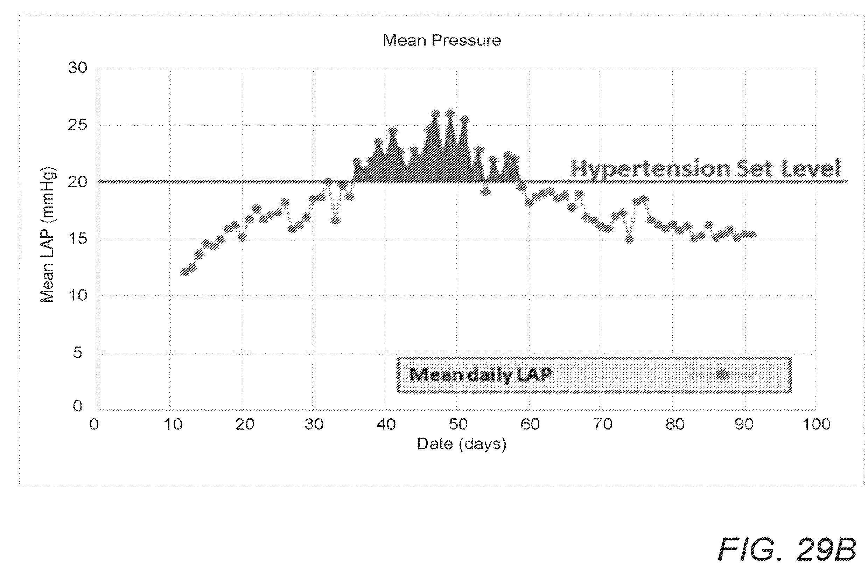

When the daily mean LAP for a particular day is greater than the hypertension level, then the difference between the daily mean LAP and the hypertension level is calculated (2840). The pulmonary congestion risk index is then calculated (2850) by summing the daily differences over the moving index time window. If the pulmonary congestion risk index exceeds the pulmonary congestion alert level, then an alert is triggered, and a treatment can be recommended (2860), and in many aspects, performed. Turning now to FIG. 29A and 29B, process 2900 is graphically illustrated on an arbitrary set of sample data in accordance with an aspect of the invention. FIG. 29A illustrates the mean LAP over time, and the newly shaded region of FIG. 29B shows the value of the pulmonary congestion risk index.

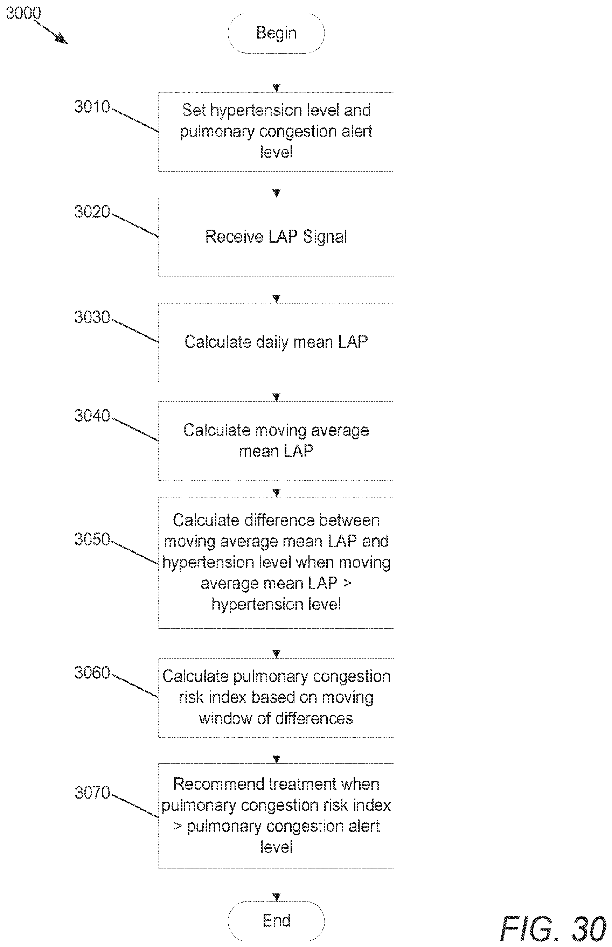

While processes like process 2800 can be used to calculate pulmonary congestion risk indices, daily measurements may jump up and down and have a level of noise associated with them. In order to reduce false alarms, daily LAP measurements can be smoothed by averaging the measurements over consecutive days. Turning now to FIG. 30, a second process for calculating pulmonary congestion risk index that uses smoothing in accordance with an aspect of the invention is illustrated.

Process 3000 includes setting (3010) the hypertension level and pulmonary congestion alert level. In numerous aspects, the hypertension level is 20mmHg, although this number can be varied depending on the particular patient as discussed above. In various aspects, the alert level is between 0 to 200 mmHg. A LAP signal is received (3020) on a daily basis, and the daily mean LAP is calculated (3030) for each day. In many aspects, the LAP signal is recorded by an atrial cardiac sensor. The LAP signal can be received at a cardiac signal processor via a transceiver as discussed above. In numerous aspects, multiple LAP signals are recorded every day and are averaged together. In numerous aspects, LAP signals are recorded daily during a similar exertion state, e.g., during rest. A moving average mean LAP is further calculated (3040). In many aspects, the moving average mean LAP is taken over a 3-day window, although a 2-10 day window can be used as well.

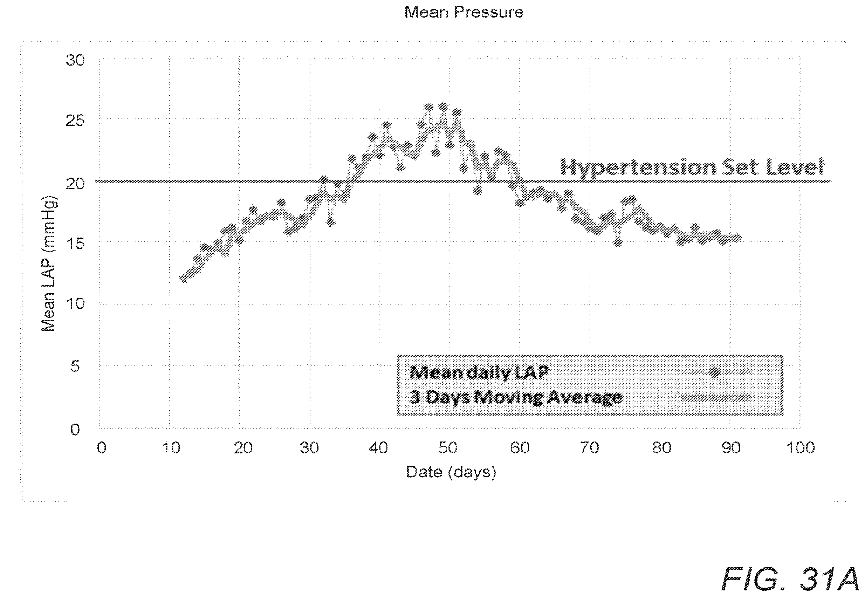

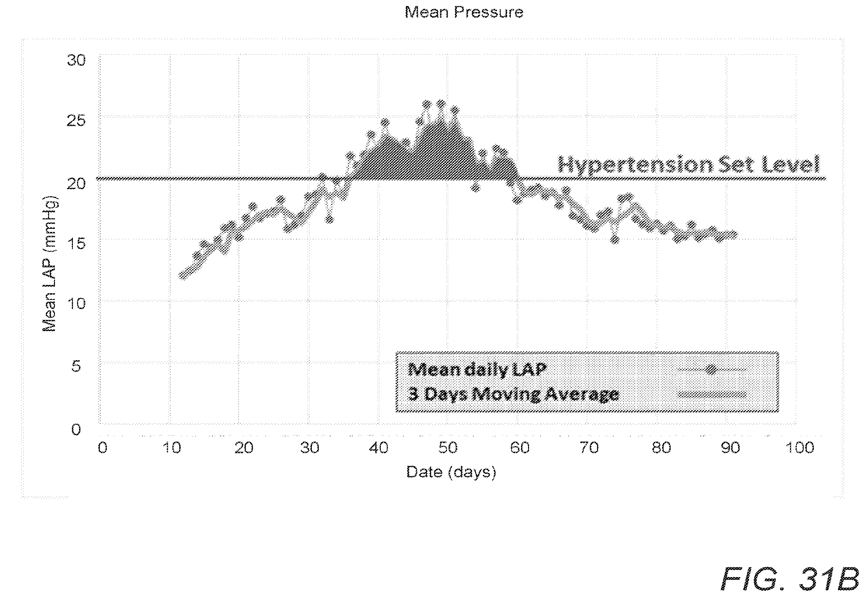

When the moving average mean LAP is greater than the hypertension level, then the difference between the moving average mean LAP and the hypertension level is calculated (3050). The pulmonary congestion risk index is then calculated (3060) by summing the differences over the index time window. If the pulmonary congestion risk index exceeds the pulmonary congestion alert level, then an alert is triggered, and a treatment can be recommended (3070), and in many aspects, performed. Turning now to FIG. 31A and 31B, process 3000 is graphically illustrated on an arbitrary set of sample data in accordance with an aspect of the invention. FIG. 31A illustrates the mean LAP over time, and the newly shaded region of FIG. 31B shows the value of the pulmonary congestion risk index.

While process 3000 introduces smoothing and can help reduce noise, day to day variability in LAP signals is often attributable to more than noise. Higher variability can indicate that the patient is less stable, and less stable patients are at a higher risk of developing pulmonary congestion with little warning. In many cases, a patient can be stable sometimes, and less stable at other times during which risk is elevated. In numerous aspects, this risk can be quantified by calculating the standard deviation of LAP relative to the smoothed LAP signal, and factor the variability into the pulmonary congestion risk index. Turning now to FIG. 3200, a third process for calculating pulmonary congestion risk index which accounts for variability in accordance with an aspect of the invention is illustrated.

Process 3200 includes setting (3210) the hypertension level and pulmonary congestion alert level. In various aspect, the alert level is 30 mmHg, however daily variability tends to add 5-10mmHg every day. This variability can be accounted for by setting the hypertension level approximately 5mmHg higher. In various aspects, the hypertension level is set at 20mmHg and the alert level is set to between 50 and 60mmHg. In numerous aspects, the hypertension level is 20mmHg, although this number can be varied depending on the particular patient as discussed above. A LAP signal is received (3220) on a daily basis, and the daily mean LAP is calculated

(3230) for each day. In many aspects, the LAP signal is recorded by an atrial cardiac sensor. The LAP signal can be received at a cardiac signal processor via a transceiver as discussed above. In numerous aspects, multiple LAP signals are recorded every day and are averaged together. In numerous aspects, LAP signals are recorded daily during a similar exertion state, e.g., during rest.

A moving N-day average mean LAP is further calculated (3240). In many aspects, the moving average mean LAP is taken over a 3-day window, N=3, although a 2-10 day window can be used as well. An M-day LAP standard deviation is calculated (3250). In many aspects, M>N. In a variety of aspects, N=3, and M=5. In numerous aspects, M = N+2.

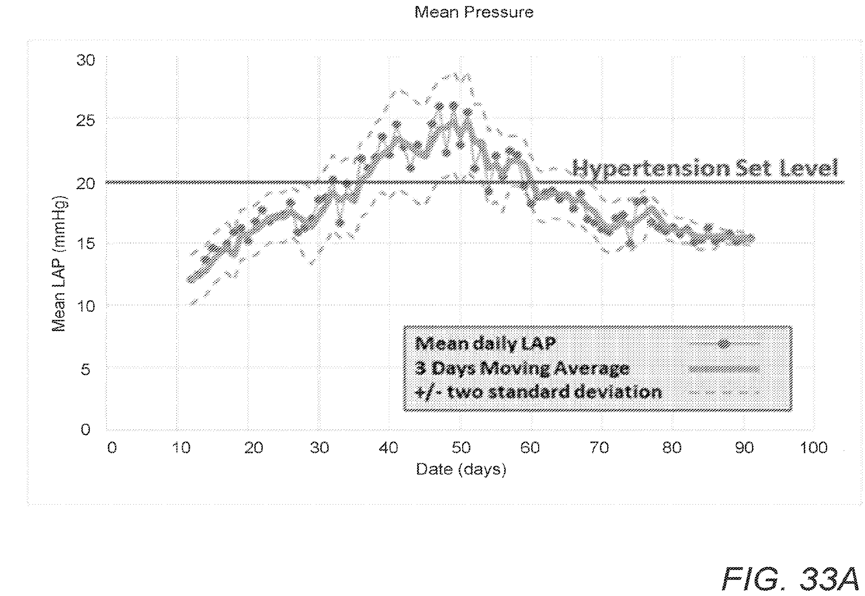

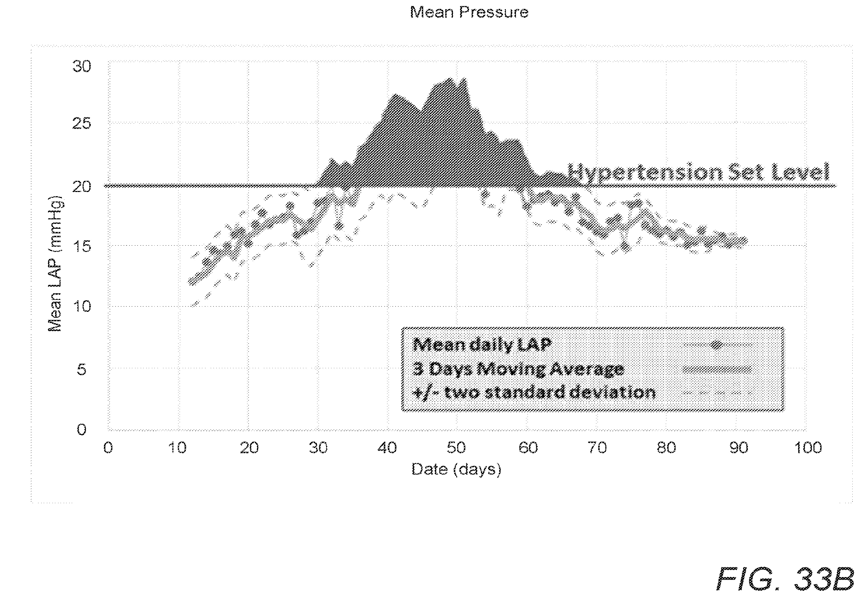

When the N-day moving average mean LAP plus 2 M-day standard deviations is greater than the hypertension level, then the difference between the moving average mean LAP + 2 M-day standard deviations and the hypertension level is calculated (3260). The pulmonary congestion risk index is then calculated (3270) by summing the differences over the index time window. If the pulmonary congestion risk index exceeds the pulmonary congestion alert level, then an alert is triggered, and a treatment can be recommended (3280), and in many aspects, performed. Turning now to FIG. 11A and 11B, process 3200 is graphically illustrated on an arbitrary set of sample data, where N=3, and M=5 in accordance with an aspect of the invention. FIG. 33A illustrates the mean LAP over time, and the newly shaded region of FIG. 33B shows the value of the pulmonary congestion risk index.

While three different specific process for calculating pulmonary congestion risk index are discussed above, as can be readily appreciated said processes can be modified with different moving windows, thresholds, and other modified parameters depending on the particular patient as appropriate to the requirements of specific applications of aspects of the invention.

F. Additional Description of Examples:

Provided below is a list of examples, each of which may include aspects of any of the other examples disclosed herein. Furthermore, aspects of any example described above may be implemented in any of the numbered examples provided below.

Example 1. A method of treating a heart condition of a patient, comprising:

obtaining a left atrial pressure (LAP) signal using an atrial cardiac sensor implanted into a left atrium of a patient;
identifying a set of A-waves and a set of V-waves in the LAP signal;
calculating an A/V ratio based on a mean pressure amplitude of the set of A-waves and a mean pressure amplitude of the set of V-waves;
determining an appropriate treatment for the patient based on the A/V ratio; and
providing the appropriate treatment to the patient.

Example 2. The method of treating a heart condition of a patient of any Example herein, in particular Example 1, identifying the set of A-waves and the set of V-waves in the LAP signal comprises decomposing the LAP signal.

Example 3. The method of treating a heart condition of a patient of any Example herein, in particular Example 2, wherein decomposing the LAP signal comprises applying a Fourier transform.

Example 4. The method of treating a heart condition of a patient of any Example herein, in particular Example 2, wherein decomposing the LAP signal comprises applying a wavelet analysis.

Example 5. The method of treating a heart condition of a patient of any Example herein, in particular Example 2, further comprising determining a respiration rate of the patient by locating a dominant frequency between 0.1 and 0.5Hz in the decomposition.

Example 6. The method of treating a heart condition of a patient of any Example herein, in particular Example 2, further comprising determining a heart rate of the patient by locating a dominant frequency between 0.8Hz and 1.7Hz in the decomposition.

Example 7. The method of treating a heart condition of a patient of any Example herein, in particular Example 2, further comprising subtracting frequency components associated with respiration rate from the LAP signal to produce a cleaned LAP signal.

Example 8. The method of treating a heart condition of a patient of any Example herein, in particular Example 7, further comprising parsing the cleaned LAP signal into a set of even waves and a set of odd waves.

Example 9. The method of treating a heart condition of any Example herein, in particular Example 8, wherein the set of even waves and the set of odd waves are exclusively labeled as a set of A-waves or a set of V-waves.

Example 10. The method of treating a heart condition of any Example herein, in particular Example 9, wherein the exclusive labeling is performed using a machine learning model trained using a training data set comprising cleaned LAP signals annotated with A-waves and V-waves.

Example 11. The method of treating a heart condition of any Example herein, in particular Example 9, wherein the exclusive labeling is performed by waveform analysis comprising identification of double peaks in A-waves.

Example 12. The method of treating a heart condition of any Example herein, in particular Example 1, wherein the appropriate treatment comprises increasing a dosage of diuretics administered to the patient by between 40% and 60% in response to the A/V ratio dropping below 0.6.

Example 13. The method of treating a heart condition of any Example herein, in particular Example 1, wherein the appropriate treatment comprises increasing a dosage of hypertension medication while maintaining a dosage of diuretics to the patient in response to a mean blood pressure of the patient increasing above 40mmHg without a change in A/V ratio.

Example 14. The method of treating a heart condition of any Example herein, in particular Example 1, wherein the appropriate treatment is a drug which is provided via automatically delivered to the patient via an infusion pump.

Example 15. A system for treating a heart condition of a patient, comprising:

a processor; and
a memory, the memory containing a cardiac monitoring application that configures the processor to:

obtain a left atrial pressure (LAP) signal recorded using an atrial cardiac sensor;
identify a set of A-waves and a set of V-waves in the LAP signal;

calculate an A/V ratio based on a mean pressure amplitude of the set of A-waves and a mean pressure amplitude of the set of V-waves; and determine an appropriate treatment for the patient based on the A/V ratio.

Example 16. The system for treating a heart condition of a patient of any Example herein, in particular Example 15, wherein to identify the set of A-waves and the set of V-waves in the LAP signal, the cardiac monitoring application further configures the processor to decompose the LAP signal.

Example 17. The system for treating a heart condition of a patient of any Example herein, in particular Example 16, wherein the decomposition comprises application of a Fourier transform.

Example 18. The system for treating a heart condition of a patient of any Example herein, in particular Example 16, wherein the decomposition comprises application of a wavelet analysis.

Example 19. The system for treating a heart condition of a patient of any Example herein, in particular Example 16, wherein the cardiac monitoring application further configures the processor to determine a respiration rate of the patient by locating a dominant frequency between 0.1 and 0.5Hz in the decomposition.

Example 20. The system for treating a heart condition of a patient of any Example herein, in particular Example 16, wherein the cardiac monitoring application further configures the processor to determine a heart rate of the patient by locating a dominant frequency between 0.8Hz and 1.7Hz in the decomposition.

Example 21. The system for treating a heart condition of a patient of any Example herein, in particular Example 16, wherein the cardiac monitoring application further configures the processor to subtract frequency components associated with respiration rate from the LAP signal to produce a cleaned LAP signal.

Example 22. The system for treating a heart condition of a patient of any Example herein, in particular Example 21, wherein the cardiac monitoring application further configures the processor to parse the cleaned LAP signal into a set of even waves and a set of odd waves.

Example 23. The system for treating a heart condition of any Example herein, in particular Example 22, wherein the set of even waves and the set of odd waves are exclusively labeled as a set of A-waves or a set of V-waves.

Example 24. The system for treating a heart condition of any Example herein, in particular Example 23, wherein the exclusive labeling is performed using a machine learning model trained using a training data set comprising cleaned LAP signals annotated with A-waves and V-waves.

Example 25. The system for treating a heart condition of any Example herein, in particular Example 23, wherein the exclusive labeling is performed by waveform analysis comprising identification of double peaks in A-waves.

Example 26. The system for treating a heart condition of any Example herein, in particular Example 15, wherein the appropriate treatment comprises increasing a dosage of diuretics administered to the patient by between 40% and 60% in response to the A/V ratio dropping below 0.6; and wherein the cardiac monitoring application further configures the processor to deliver the dosage of diuretics via an infusion pump communicatively coupled to the processor.

Example 27. The system for treating a heart condition of any Example herein, in particular Example 15, wherein the appropriate treatment comprises increasing a dosage of hypertension medication while maintaining a dosage of diuretics to the patient in response to a mean blood pressure of the patient increasing above 40mmHg without a change in A/V ratio; and wherein the cardiac monitoring application further configures the processor to deliver the dosage of diuretics via an infusion pump communicatively coupled to the processor.

Example 28. The system for treating a heart condition of any Example herein, in particular Example 15, wherein the appropriate treatment is a drug which is provided via automatically delivered to the patient via an infusion pump communicatively coupled to the processor.

Example 29. A cardiac monitoring system for calculating A/V ratio, comprising:

an atrial cardiac sensor configured to be implanted in a left atrium of a patient to record a left atrial pressure (LAP) signal;
a transceiver configured to:

wirelessly provide power to the atrial cardiac sensor; and
receive the LAP signal from the atrial cardiac sensor;

a processor; and
a memory containing a cardiac monitoring application, where the cardiac monitoring application directs the processor to:

identify a set of A-waves and a set of V-waves in the LAP signal; and
calculate an A/V ratio based upon a mean pressure amplitude of the set of A-waves and a mean pressure amplitude of the set of V-waves.

Example 30. The cardiac monitoring system of any Example herein, in particular Example 29, wherein to identify the set of A-waves and the set of V-waves in the LAP signal, the cardiac monitoring application further configures the processor to decompose the LAP signal.

Example 31. The cardiac monitoring system of any Example herein, in particular Example 30, wherein the decomposition comprises application of a Fourier transform.

Example 32. The cardiac monitoring system of any Example herein, in particular Example 30, wherein the decomposition comprises application of a wavelet analysis.

Example 33. The cardiac monitoring system of any Example herein, in particular Example 30, wherein the cardiac monitoring application further configures the processor to determine a respiration rate of the patient by locating a dominant frequency between 0.1 and 0.5Hz in the decomposition.

Example 34. The cardiac monitoring system of any Example herein, in particular Example 30, wherein the cardiac monitoring application further configures the processor to determine a heart rate of the patient by locating a dominant frequency between 0.8Hz and 1.7Hz in the decomposition.

Example 35. The cardiac monitoring system of any Example herein, in particular Example 30, wherein the cardiac monitoring application further configures the processor to subtract frequency components associated with respiration rate from the LAP signal to produce a cleaned LAP signal.

Example 36. The cardiac monitoring system of any Example herein, in particular Example 35, wherein the cardiac monitoring application further configures the processor to parse the cleaned LAP signal into a set of even waves and a set of odd waves.

Example 37. The cardiac monitoring system of any Example herein, in particular Example 36, wherein the set of even waves and the set of odd waves are exclusively labeled as a set of A-waves or a set of V-waves.

Example 38. The cardiac monitoring system of any Example herein, in particular Example 37, wherein the exclusive labeling is performed using a machine learning model trained using a training data set comprising cleaned LAP signals annotated with A-waves and V-waves.

Example 39. The cardiac monitoring system of any Example herein, in particular Example 37, wherein the exclusive labeling is performed by waveform analysis comprising identification of double peaks in A-waves.

Example 40. A method of calculating A/V ratio, comprising:

obtain a left atrial pressure (LAP) signal using an atrial cardiac sensor configured to be implanted in a left atrium of a patient;
identify a set of A-waves and a set of V-waves in the LAP signal; and
calculate an A/V ratio based upon a mean pressure amplitude of the set of A-waves and a mean pressure amplitude of the set of V-waves.

Example 41. The method of calculating A/V ratio of any Example herein, in particular Example 40, wherein to identify the set of A-waves and the set of V-waves in the LAP signal, the cardiac monitoring application further configures the processor to decompose the LAP signal.

Example 42. The method of calculating A/V ratio of any Example herein, in particular Example 41, wherein the decomposition comprises application of a Fourier transform.

Example 43. The method of calculating A/V ratio of any Example herein, in particular Example 41, wherein the decomposition comprises application of a wavelet analysis.

Example 44. The method of calculating A/V ratio of any Example herein, in particular Example 41, wherein the cardiac monitoring application further configures the processor to determine a respiration rate of the patient by locating a dominant frequency between 0.1 and 0.5Hz in the decomposition.

Example 45. The method of calculating A/V ratio of any Example herein, in particular Example 41, wherein the cardiac monitoring application further configures the processor to determine a heart rate of the patient by locating a dominant frequency between 0. 8Hz and 1.7Hz in the decomposition.

Example 46. The method of calculating A/V ratio of any Example herein, in particular Example 41, wherein the cardiac monitoring application further configures the processor to subtract frequency components associated with respiration rate from the LAP signal to produce a cleaned LAP signal.

Example 47. The method of calculating A/V ratio of any Example herein, in particular Example 46, wherein the cardiac monitoring application further configures the processor to parse the cleaned LAP signal into a set of even waves and a set of odd waves.

Example 48. The method of calculating A/V ratio of any Example herein, in particular Example 47, wherein the set of even waves and the set of odd waves are exclusively labeled as a set of A-waves or a set of V-waves.

Example 49. The method of calculating A/V ratio of any Example herein, in particular Example 48, wherein the exclusive labeling is performed using a machine learning model trained using a training data set comprising cleaned LAP signals annotated with A-waves and V-waves

Example 50. The method of calculating A/V ratio of any Example herein, in particular Example 48, wherein the exclusive labeling is performed by waveform analysis comprising identification of double peaks in A-waves.

Example 51. A method of monitoring patient response to exertion, comprising:

recording a first blood pressure signal during a rest state of a patient using an atrial cardiac sensor implanted into the left atrium of the patient;
detecting a change from a rest state to an exertion state;

recording a second blood pressure signal during the exertion state in response to the detection of the change in states of the patient using the atrial cardiac sensor,

receiving the first and second blood pressure signals at a cardiac signal processor;

calculating a first plurality of cardiac metrics based on the first blood pressure signal using the cardiac signal processor;

calculating a second plurality of cardiac metrics based on the second blood pressure signals using the cardiac signal processor;

determining a change in a health state of the patient based on a change between the first and second plurality of cardiac metrics using the cardiac signal processor; and

treating the patient in response to the change in the health state.

Example 52. The method of monitoring patient response to exertion of any Example herein, in particular Example 51, wherein a transceiver directs the atrial cardiac sensor to perform the recording of the first and second blood pressure signal.

Example 53. The method of monitoring patient response to exertion of any Example herein, in particular Example 51, wherein the first blood pressure signal is obtained while the patient is at rest.

Example 54. The method of monitoring patient response to exertion of any Example herein, in particular Example 51, wherein the second blood pressure signal is obtained while the patient is in an active state.

Example 55. The method of monitoring patient response to exertion of any Example herein, in particular Example 51, wherein the first and second blood pressure signals are left atrial pressure signals.

Example 56. The method of monitoring patient response to exertion of any Example herein, in particular Example 51, wherein the first plurality of cardiac metrics and the second plurality of cardiac metrics comprise an A/V ratio.

Example 57. The method of monitoring patient response to exertion of any Example herein, in particular Example 51, wherein the first plurality of cardiac metrics and the second plurality of cardiac metrics comprise a respiration rate.

Example 58. The method of monitoring patient response to exertion of any Example herein, in particular Example 51, wherein the first plurality of cardiac metrics and the second plurality of cardiac metrics comprise a heart rate.

Example 59. The method of monitoring patient response to exertion of any Example herein, in particular Example 51, wherein the treatment is automated delivery of a drug via an infusion pump.

Example 60. A cardiac patient exertion monitoring system, comprising:

an atrial cardiac sensor configured to be implanted in a left atrium of a patient to record:

a first blood pressure signal during a rest state of the patient; and
a second blood pressure signal during an exertion state of the patient; and

a cardiac signal processor configured to:

receive the first and second blood pressure signals;
calculate a first plurality of cardiac metrics based on the first blood pressure signal;
calculate a second plurality of cardiac metrics based on the second blood pressure signals; and
provide a warning indicating a health state of the patient based on a change between the first and second plurality of cardiac metrics.

Example 61. The cardiac patient exertion monitoring system of any Example herein, in particular Example 60, further comprising a transceiver configured to:

obtain the first and second blood pressure signals from the atrial cardiac sensor; and
transmit the first and second blood pressure signals to the cardiac signal processor.

Example 62. The cardiac patient exertion monitoring system of any Example herein, in particular Example 61, wherein the transceiver is further configured to power the atrial cardiac sensor.

Example 63. The cardiac patient exertion monitoring system of any Example herein, in particular Example 60, wherein powering the atrial cardiac sensor triggers the atrial cardiac sensor to record the first cardiac signal.

Example 64. The cardiac patient exertion monitoring system of any Example herein, in particular Example 60, further comprising an infusion pump; and wherein the cardiac signal processor is further configured to automatically deliver a drug to the patient via the infusion pump based on the health state of the patient.

Example 65. The cardiac patient exertion monitoring system of any Example herein, in particular Example 60, wherein the first and second blood pressure signals are left atrial pressure signals.

Example 66. The cardiac patient exertion monitoring system of any Example herein, in particular Example 60, wherein the first plurality of cardiac metrics and the second plurality of cardiac metrics comprise an A/V ratio.

Example 67. The cardiac patient exertion monitoring system of any Example herein, in particular Example 60, wherein the first plurality of cardiac metrics and the second plurality of cardiac metrics comprise a respiration rate.

Example 68. The cardiac patient exertion monitoring system of any Example herein, in particular Example 60, wherein the first plurality of cardiac metrics and the second plurality of cardiac metrics comprise a heart rate.

Example 69. A cardiac patient exertion monitoring system, comprising:

an atrial cardiac sensor configured to be implanted in a left atrium of a patient to record:

a first blood pressure signal during a rest state of the patient; and
a second blood pressure signal during an exertion state of the patient; and

a cardiac signal processor configured to:

receive the first and second blood pressure signals;
calculate a first heart rate (HR), a first mean blood pressure (P), a first respiration rate (RR), and a first A/V ratio based on the first blood pressure signal;
calculate a second HR, a second P, a second RR, and a second A/V ratio based on the second blood pressure signal;
calculate a P/HR ratio between the change between the first P and the second P, and the change between the first HR and the second HR;
calculate a RR/HR ratio between the change between the first RR and the second RR, and the change between the first HR and the second HR;
calculate an (A/V)/HR ratio between the change between the first A/V ratio and the second A/V ratio, and the change between the first HR and the second HR; and
provide a warning indicating a negative health state of the when at least one of the P/HR ratio, the RR/HR ratio, and the (A/V)/HR ratio exceed a respective threshold value.

Example 70. The cardiac patient exertion monitoring system of any Example herein, in particular Example 69, further comprising an infusion pump; and wherein the cardiac signal processor is further configured to automatically infuse the patient with a drug in order to treat a heart condition in response to the negative health state.

Example 71. A cardiac patient exertion monitoring system, comprising:

an atrial cardiac sensor configured to be implanted in a left atrium of a patient to record a plurality pressure signals over the course of at least one day; and

a cardiac signal processor configured to:

receive the plurality of pressure signals;
calculate, for each pressure signal in the plurality of pressure signals, a heart rate (HR) and an A/V ratio;
plot a curve where A/V ratio is on the y-axis and recording time is on the x-axis;
identify times during which the patient was in an exertion state and times during which the patient was in a resting state based on the HRs;
annotate the curve with identified exertion states and resting states; and
display the curve.

Example 72. The cardiac patient exertion monitoring system of any Example herein, in particular Example 71, wherein the cardiac signal processor is further configured to:

calculate, for each pressure signal in the plurality of pressure signals, a mean blood pressure (P);
calculate, for each pair of successive pressure signals in the plurality of pressure signals, a P/HR;
plot an additional curve where P/HR ratio is on the y-axis and recording time is on the x-axis;
annotate the additional curve with identified exertion states and resting states; and display the additional curve.

Example 73. The cardiac patient exertion monitoring system of any Example herein, in particular Example 71, wherein the cardiac signal processor is further configured to:

calculate, for each pair of successive pressure signals in the plurality of pressure signals, an (A/V)/HR;
plot an additional curve where (A/V)/HR ratio is on the y-axis and recording time is on the x-axis;
annotate the additional curve with identified exertion states and resting states; and
display the additional curve.

Example 74. The cardiac patient exertion monitoring system of any Example herein, in particular Example 71, wherein the cardiac signal processor is further configured to provide a warning indicating a negative health state based on the curve.

Example 75. A method of monitoring cardiac patient exertion, comprising:

receiving a plurality of pressure signals from a cardiac sensor implanted in the left atrium of a cardiac patient's heart;
calculating, for each pressure signal in the plurality of pressure signals, a heart rate (HR) and an A/V ratio;
plotting a curve where A/V ratio is on the y-axis and recording time is on the x-axis;

identifying times during which the patient was in an exertion state and times during which the patient was in a resting state based on the HRs;

annotating the curve with identified exertion states and resting states; and

displaying the curve.

Example 76. The method of monitoring cardiac patient exertion of any Example herein, in particular Example 75, further comprising:

calculating, for each pressure signal in the plurality of pressure signals, a mean blood pressure (P);

calculating, for each pair of successive pressure signals in the plurality of pressure signals, a P/HR;

plotting an additional curve where P/HR ratio is on the y-axis and recording time is on the x-axis;

annotating the additional curve with identified exertion states and resting states; and

displaying the additional curve.

Example 77. The method of monitoring cardiac patient exertion of any Example herein, in particular Example 75, further comprising:

calculating, for each pair of successive pressure signals in the plurality of pressure signals, an (A/V)/HR;

plotting an additional curve where (A/V)/HR ratio is on the y-axis and recording time is on the x-axis;

annotating the additional curve with identified exertion states and resting states; and

displaying the additional curve.

Example 78. The method of monitoring cardiac patient exertion of any Example herein, in particular Example 75, wherein the cardiac signal processor is further configured to provide a warning indicating a negative health state based on the curve.

Example 79. A method for differently presenting health metrics on a personalized, per-patient basis to a health professional, comprising:

receiving a left atrial blood pressure waveform from an atrial cardiac sensor implanted in the left atrium of the patient;

obtaining patient background medical information, where the patient background medical information comprises a value for a plurality of categorical parameters regarding a patient;

generating a parameterized patient profile based on the patient background information;

classifying the patient based on the parametrized patient profile;

calculating cardiac metrics comprising: mean left atrial pressure (LAP), peak LAP, heart rate, respiration rate, A/V ratio, and exertion response based on the left atrial blood pressure waveform; and

displaying the cardiac metrics based on the classification such that an indicator is provided as to whether or not each cardiac metric is within a normal range for the classification.

Example 80. The method of any Example herein, in particular Example 79, wherein the parameterized patient profile comprises a parameter for:

sex;

cardiac condition;

pulmonary hypertension group;

arrhythmia;

mitral regurgitation;

ischemia scar tissue; and

pacemaker implantation.

Example 81. The method of any Example herein, in particular Example 79, wherein each unique combination of parameters in the parameterized patient profile is associated with a class.

Example 82. The method of any Example herein, in particular Example 79, wherein to classify the patient, the patient profile is provided to a machine learning model configured to provide a classification.

Example 83. The method of any Example herein, in particular Example 82, wherein the machine learning model is a supervised machine learning model.

Example 84. The method of any Example herein, in particular Example 83, wherein the machine learning model is a random forest model.

Example 85. The method of any Example herein, in particular Example 83, wherein the machine learning model is a support vector machine.

Example 86. The method of any Example herein, in particular Example 83, wherein the supervised machine learning model trained using a training data set comprising patient profiles from an electronic health record system annotated with patient outcomes.

Example 87. The method of any Example herein, in particular Example 82, wherein the machine learning model is an unsupervised clustering model, and the unsupervised clustering model is provided with patient profiles from an electronic health record system and the patient profile in order to classify the patient as belonging to a particular cluster.

Example 88. The method of any Example herein, in particular Example 87, wherein the normal range for cardiac metrics for the particular cluster is derived from cardiac metric values from electronic health records for patients in the particular cluster at time of discharge.

Example 89. An electronic health record system for differentially presenting health metrics on a personalized basis, comprising:

a processor; and

a memory, the memory containing an application that configures the processor to:

receive a left atrial blood pressure waveform from an atrial cardiac sensor implanted in the left atrium of the patient;

obtain patient background medical information, where the patient background medical information comprises a value for a plurality of categorical parameters regarding a patient;

generate a parameterized patient profile based on the patient background information;

classify the patient based on the parametrized patient profile;

calculate cardiac metrics comprising: mean left atrial pressure (LAP), peak LAP, heart rate, respiration rate, A/V ratio, and exertion response based on the left atrial blood pressure waveform; and

display the cardiac metrics based on the classification such that an indicator is provided as to whether or not each cardiac metric is within a normal range for the classification.

Example 90. The system of any Example herein, in particular Example 89, wherein the parameterized patient profile comprises a parameter for:

sex;
cardiac condition;
pulmonary hypertension group;
arrhythmia;
mitral regurgitation;
ischemia scar tissue; and
pacemaker implantation.

Example 91. The system of any Example herein, in particular Example 89, wherein each unique combination of parameters in the parameterized patient profile is associated with a class.

Example 92. The system of any Example herein, in particular Example 89, wherein to classify the patient, the application further directs the processor to provide the patient profile to a machine learning model configured to provide a classification.

Example 93. The system of any Example herein, in particular Example 92, wherein the machine learning model is a supervised machine learning model.

Example 94. The system of any Example herein, in particular Example 93, wherein the machine learning model is a random forest model.

Example 95. The system of any Example herein, in particular Example 93, wherein the machine learning model is a support vector machine.

Example 96. The system of any Example herein, in particular Example 93, wherein the supervised machine learning model trained using a training data set comprising patient profiles from an electronic health record system annotated with patient outcomes.

Example 97. The system of any Example herein, in particular Example 92, wherein the machine learning model is an unsupervised clustering model, and the unsupervised clustering model is provided with patient profiles from an electronic health record system and the patient profile in order to classify the patient as belonging to a particular cluster.

Example 98. The system of any Example herein, in particular Example 97, wherein the normal range for cardiac metrics for the particular cluster is derived from cardiac metric values from electronic health records for patients in the particular cluster at time of discharge.

Example 99. A method of treating a heart condition of a patient, comprising:

obtaining a left atrial pressure (LAP) signal using an atrial cardiac sensor implanted into a left atrium of a patient;

identifying a set of A-waves and a set of V-waves in the LAP signal;

calculating an A/V ratio based on a mean pressure amplitude of the set of A-waves and a mean pressure amplitude of the set of V-waves;

calculating an average maximum amplitude of the set of A-waves;

calculating an average maximum amplitude of the set of V-waves;

calculating an average minimum amplitude of inflection points at the transition of A-waves to V-waves in the LAP signal;

calculating an average minimum amplitude of inflection points at the transition of V-waves to A-waves in the LAP signal;

calculating a difference between the average maximum amplitude of the set of A-waves and the average maximum amplitude of the set of V-waves, $\Delta$Max;

calculating a difference between the average minimum amplitude of inflection points at the transition of A-waves to V-waves and the average minimum amplitude of inflection points at the transition of V-waves to A-waves, $\Delta$Min;

calculating a mitral valve index (MV1) based on the A/V ratio, $\Delta$Max, AMin; and

determining a treatment for the patient based on the MVI exceeding a threshold value.

Example 100. The method of any Example herein, in particular Example 99, wherein the treatment is the implantation of an artificial heart valve.

Example 101. The method of any Example herein, in particular Example 99, wherein the treatment is the replacement of an artificial heart valve.

Example 102. The method of any Example herein, in particular Example 99, wherein the appropriate treatment is a surgical percutaneous valve repair.

Example 103. The method of any Example herein, in particular Example 99, wherein MVI is calculated as $\left( A/V\,Ratio \right)\left( \frac{C1}{\Delta Max} \right)\left( \frac{C2}{\Delta Min} \right)$, C1 is the $\Delta$Max

of a healthy heart, and C2 is the ΔMin of a healthy heart.

Example 104. The method of any Example herein, in particular Example 103, wherein C1 and C2 are between 1 and 5.

Example 105. The method of any Example herein, in particular Example 104, wherein the threshold is 1, and an MVI > 1 indicates a healthy patient where no treatment is required.

Example 106. The method of any Example herein, in particular Example 99, further comprising providing the determined treatment.

Example 107. A mitral valve monitoring and treatment system, comprising:

an atrial cardiac sensor configured to be implanted in a left atrium of a patient to record a left atrial pressure (LAP) signal;

a transceiver configured to:

wirelessly provide power to the atrial cardiac sensor; and
receive the LAP signal from the atrial cardiac sensor;

a processor; and
a memory containing a cardiac monitoring application, where the cardiac monitoring application directs the processor to:

identify a set of A-waves and a set of V-waves in the LAP signal;
calculate an A/V ratio based upon a mean pressure amplitude of the set of A-waves and a mean pressure amplitude of the set of V-waves;
calculate an average maximum amplitude of the set of A-waves;
calculate an average maximum amplitude of the set of V-waves;
calculate an average minimum amplitude of inflection points at the transition of A-waves to V-waves in the LAP signal;
calculate an average minimum amplitude of inflection points at the transition of V-waves to A-waves in the LAP signal;
calculate a difference between the average maximum amplitude of the set of A-waves and the average maximum amplitude of the set of V-waves, ΔMax;
calculate a difference between the average minimum amplitude of inflection points at the transition of A-waves to V-waves and the average minimum amplitude of inflection points at the transition of V-waves to A-waves, ΔMin;
calculate a mitral valve index (MVI) based on the A/V ratio, ΔMax, ΔMin; and
determine a treatment for the patient based on the MVI exceeding a threshold value.

Example 108. The system of any Example herein, in particular Example 107, wherein the treatment is the implantation of an artificial heart valve.

Example 109. The system of any Example herein, in particular Example 107, wherein the treatment is the replacement of an artificial heart valve.

Example 110. The system of any Example herein, in particular Example 107, wherein the treatment is a surgical percutaneous valve repair.

Example 111. The system of any Example herein, in particular Example 107, wherein MVI is calculated as $(A/V\,Ratio)\left(\frac{C1}{\Delta Max}\right)\left(\frac{C2}{\Delta Min}\right)$, C1 is the AMax of a healthy heart, and C2 is the ΔMin of a healthy heart.

Example 112. The system of any Example herein, in particular Example 111, wherein C1 and C2 are between 1 and 5.

Example 113. The system of any Example herein, in particular Example 107, wherein the threshold is 1, and an MVI > 1 indicates a healthy patient where no treatment is required.

Example 114. An artificial heart valve, comprising:

a sensor configured to record a left atrial pressure (LAP) signal; and
a transmitter configured to wirelessly transmit the LAP signal to an external monitoring device;
where the monitoring device is configured to determine if the artificial heart valve requires replacement based on the LAP signal.

Example 115. The artificial heart valve of any Example herein, in particular Example 114, wherein to determine if the artificial heart valve requires replacement, the monitoring device is further configured to:

identify a set of A-waves and a set of V-waves in the LAP signal;
calculate an A/V ratio based upon a mean pressure amplitude of the set of A-waves and a mean pressure amplitude of the set of V-waves;
calculate an average maximum amplitude of the set of A-waves;
calculate an average maximum amplitude of the set of V-waves;
calculate an average minimum amplitude of inflection points at the transition of A-waves to V-waves in the LAP signal;
calculate an average minimum amplitude of inflection points at the transition of V-waves to A-waves in the LAP signal;
calculate a difference between the average maximum amplitude of the set of A-waves and the average maximum amplitude of the set of V-waves, ΔMax;
calculate a difference between the average minimum amplitude of inflection points at the transition of A-waves to V-waves and the average minimum amplitude of inflection points at the transition of V-waves to A-waves, ΔMin;

calculate a mitral valve index (MVI) based on the A/V ratio, ΔMax, ΔMin; and

determine that the artificial heart valve requires replacement when MVI < 1.

Example 116. The artificial heart valve of any Example herein, in particular Example 114, wherein MVI is calculated as $(A/V Ratio)(\frac{C1}{\Delta Max})(\frac{C2}{\Delta Min})$, C1 is the ΔMax of a healthy heart, and C2 is the ΔMin of a healthy heart.

Example 117. The artificial heart valve of any Example herein, in particular Example 115, wherein C1 and C2 are between 1 and 5.

Example 118. A method of monitoring pulmonary congestion of a patient, comprising:

obtaining a plurality of left atrial pressure (LAP) signals using an atrial cardiac sensor implanted into a left atrium of a patient, where each LAP is obtained on a different day in a plurality of days;

calculating a daily mean LAP for each given day in the plurality of days based on the LAP signal obtained on the given day;

calculating the difference between the daily mean LAP and a hypertension level when the daily mean LAP is greater than the hypertension level value for the given day; and

calculating a pulmonary congestion risk index based on the calculated difference.

Example 119. The method of any Example herein, in particular Example 118, wherein the days in the plurality of days are consecutive.

Example 120. The method of any Example herein, in particular Example 118, further comprising recommending a treatment when the pulmonary congestion risk index is greater than an alert level.

Example 121. The method of any Example herein, in particular Example 120, wherein the hypertension level is 20mmHg, and the alert level is 30mmHg.

Example 122. The method of any Example herein, in particular Example 120, wherein the recommended treatment is mitral valve replacement.

Example 123. The method of any Example herein, in particular Example 120, wherein the recommended treatment is mitral valve repair.

Example 124. A method of monitoring pulmonary congestion of a patient, comprising:

obtaining a plurality of left atrial pressure (LAP) signals using an atrial cardiac sensor implanted into a left atrium of a patient, where each LAP is obtained on a different day in a plurality of days;

calculating a daily mean LAP for each given day in the plurality of days based on the LAP signal obtained on the given day;

calculating a moving average mean LAP based on a number of consecutive most recent daily mean LAP values;

calculating the difference between the moving average mean LAP and a hypertension level when the moving average mean LAP is greater than the hypertension level value for the given day; and

calculating a pulmonary congestion risk index based on the calculated differences.

Example 125. The method of any Example herein, in particular Example 124, wherein the days in the plurality of days are consecutive.

Example 126. The method of any Example herein, in particular Example 118, further comprising recommending a treatment when the pulmonary congestion risk index is greater than an alert level.

Example 127. The method of any Example herein, in particular Example 120, wherein the hypertension level is 20mmHg, and the alert level is between 0 and 200 mmHg.

Example 128. The method of any Example herein, in particular Example 126, wherein the recommended treatment is mitral valve replacement.

Example 129. The method of any Example herein, in particular Example 126, wherein the recommended treatment is mitral valve repair.

Example 130. The method of any Example herein, in particular Example 124, wherein the number of consecutive most recent daily mean LAP values is between 2 and 10.

Example 131. A method of monitoring pulmonary congestion of a patient, comprising:

obtaining a plurality of left atrial pressure (LAP) signals using an atrial cardiac sensor implanted into a left atrium of a patient, where each LAP is obtained on a different day in a plurality of days;

calculating a daily mean LAP for each given day in the plurality of days based on the LAP signal obtained on the given day;

calculating a moving average mean LAP based on a first number of consecutive most recent daily mean LAP values;

calculating a LAP standard deviation based on a second number of consecutive most recent daily mean LAP values; and

calculating the difference between the moving average mean LAP plus two LAP standard deviations and a hypertension level when the moving average mean LAP plus two LAP standard deviations is greater than the hypertension level value for the given day; and

calculating a pulmonary congestion risk index based on the calculated differences.

Example 132. The method of any Example herein, in particular Example 131, wherein the days in the plurality of days are consecutive.

Example 133. The method of any Example herein, in particular Example 131, further comprising recommending a treatment when the pulmonary congestion risk index is greater than an alert level.

Example 134. The method of any Example herein, in particular Example 133, wherein the hypertension level is 20mmHg, and the alert level is between 50 and 60mmHg.

Example 135. The method of any Example herein, in particular Example 133, wherein the recommended treatment is mitral valve replacement.

Example 136. The method of any Example herein, in particular Example 133, wherein the recommended treatment is mitral valve repair.

Example 137. The method of any Example herein, in particular Example 131, wherein the first and numbers of consecutive most recent daily mean LAP values are between 2 and 10, and the first number is less than the second number.

Example 138. A system for monitoring pulmonary congestion of a patient, comprising:

an atrial cardiac sensor configured to be implanted in a left atrium of a patient to record a plurality of left atrial pressure (LAP) signals, where each LAP signal in the plurality of LAP signals is obtained on a different day in a plurality of days;
a transceiver configured to:

wirelessly provide power to the atrial cardiac sensor; and
receive the LAP signal from the atrial cardiac sensor;

a processor; and
a memory containing a cardiopulmonary monitoring application, where the cardiopulmonary monitoring application directs the processor to:

obtain the plurality LAP signals;
calculate a daily mean LAP for each given day in the plurality of days based on the LAP signal obtained on the given day;
calculate the difference between the daily mean LAP and a hypertension level when the daily mean LAP is greater than the hypertension level value for the given day; and
calculate a pulmonary congestion risk index based on the calculated difference.

Example 139. The system of any Example herein, in particular Example 138, wherein the days in the plurality of days are consecutive.

Example 140. The system of any Example herein, in particular Example 138, wherein the cardiopulmonary monitoring application further directs the processor to recommend a treatment when the pulmonary congestion risk index is greater than an alert level.

Example 141. The system of any Example herein, in particular Example 140, wherein the hypertension level is 20mmHg, and the alert level is 30mmHg.

Example 142. The system of any Example herein, in particular Example 140, wherein the recommended treatment is mitral valve replacement.

Example 143. The system of any Example herein, in particular Example 140, wherein the recommended treatment is mitral valve repair.

Example 144. A system for monitoring pulmonary congestion of a patient, comprising:

an atrial cardiac sensor configured to be implanted in a left atrium of a patient to record a plurality of left atrial pressure (LAP) signals, where each LAP signal in the plurality of LAP signals is obtained on a different day in a plurality of days;
a transceiver configured to:

wirelessly provide power to the atrial cardiac sensor; and
receive the LAP signal from the atrial cardiac sensor;

a processor; and
a memory containing a cardiopulmonary monitoring application, where the cardiopulmonary monitoring application directs the processor to:

obtain the plurality LAP signals;
calculate a daily mean LAP for each given day in the plurality of days based on the LAP signal obtained on the given day;
calculate a moving average mean LAP based on a number of consecutive most recent daily mean LAP values;
calculate the difference between the moving average mean LAP and a hypertension level when the moving average mean LAP is greater than the hypertension level value for the given day; and
calculate a pulmonary congestion risk index based on the calculated differences.

Example 145. The system of any Example herein, in particular Example 144, wherein the days in the plurality of days are consecutive.

Example 146. The system of any Example herein, in particular Example 144, wherein the cardiopulmonary monitoring application further directs the processor to recommend a treatment when the pulmonary congestion risk index is greater than an alert level.

Example 147. The system of any Example herein, in particular Example 146, wherein the hypertension level is 20mmHg, and the alert level is between 0 and 200 mmHg.

Example 148. The system of any Example herein, in particular Example 146, wherein the recommended treatment is mitral valve replacement.

Example 149. The system of any Example herein, in particular Example 146, wherein the recommended treatment is mitral valve repair.

Example 150. The system of any Example herein, in particular Example 124, wherein the number of consecutive most recent daily mean LAP values is between 2 and 10.

Example 151. A system for monitoring pulmonary congestion of a patient, comprising:

an atrial cardiac sensor configured to be implanted in a left atrium of a patient to record a plurality of left atrial pressure (LAP) signals, where each LAP signal in the plurality of LAP signals is obtained on a different day in a plurality of days;
a transceiver configured to:

wirelessly provide power to the atrial cardiac sensor; and
receive the LAP signal from the atrial cardiac sensor;

a processor; and
a memory containing a cardiopulmonary monitoring application, where the cardiopulmonary monitoring application directs the processor to:

obtain the plurality LAP signals;
calculate a daily mean LAP for each given day in the plurality of days based on the LAP signal obtained on the given day;
calculate a moving average mean LAP based on a first number of consecutive most recent daily mean LAP values;
calculate a LAP standard deviation based on a second number of consecutive most recent daily mean LAP values;
calculate the difference between the moving average mean LAP plus two LAP standard deviations and a hypertension level when the moving average mean LAP plus two LAP standard deviations is greater than the hypertension level value for the given day; and

calculate a pulmonary congestion risk index based on the calculated differences.

Example 152. The system of any Example herein, in particular Example 151, wherein the days in the plurality of days are consecutive.

Example 153. The system of any Example herein, in particular Example 151, wherein the cardiopulmonary monitoring application further directs the processor to recommend a treatment when the pulmonary congestion risk index is greater than an alert level.

Example 154. The system of any Example herein, in particular Example 153, the hypertension level is 20mmHg, and the alert level is between 50 and 60mmHg.

Example 155. The system of any Example herein, in particular Example 153, wherein the recommended treatment is mitral valve replacement.

Example 156. The system of any Example herein, in particular Example 153, wherein the recommended treatment is mitral valve repair.

Example 157. The system of any Example herein, in particular Example 151, wherein the first and numbers of consecutive most recent daily mean LAP values are between 2 and 10, and the first number is less than the second number.

Although specific systems and methods for cardiac health monitoring and treatment are discussed above, many different systems and methods can be implemented in accordance with many different aspects of the invention. It is therefore to be understood that the present invention may be practiced in ways other than specifically described, without departing from the scope and spirit of the present invention. Thus, aspects of the present invention should be considered in all respects as illustrative and not restrictive. Accordingly, the scope of the invention should be determined not by the aspects illustrated, but by the appended claims and their equivalents.

Examples are set out in the following list of numbered clauses:

1. A method of treating a heart condition of a patient, comprising:

obtaining a left atrial pressure (LAP) signal using an atrial cardiac sensor implanted into a left atrium of a patient;
identifying a set of A-waves and a set of V-waves in the LAP signal;
calculating an A/V ratio based on a mean pressure amplitude of the set of A-waves and a mean pressure amplitude of the set of V-waves;
determining an appropriate treatment for the patient based on the A/V ratio; and
providing the appropriate treatment to the patient.

2. The method of treating a heart condition of a patient of clause 1, identifying the set of A-waves and the set of V-waves in the LAP signal comprises decomposing the LAP signal.

3. The method of treating a heart condition of a patient of clause 2, wherein decomposing the LAP signal comprises applying a Fourier transform.

4. The method of treating a heart condition of a patient of clause 2, wherein decomposing the LAP signal comprises applying a wavelet analysis.

5. The method of treating a heart condition of a patient of clause 2, further comprising determining a respiration rate of the patient by locating a dominant frequency between 0.1 and 0.5Hz in the decomposition.

6. The method of treating a heart condition of a patient of clause 2, further comprising determining a heart rate of the patient by locating a dominant frequency between 0.8Hz and 1.7Hz in the decomposition.

7. The method of treating a heart condition of a patient of clause 2, further comprising subtracting frequency components associated with respiration rate from the LAP signal to produce a cleaned LAP signal.

8. The method of treating a heart condition of a patient of clause 7, further comprising parsing the cleaned LAP signal into a set of even waves and a set of odd waves.

9. The method of treating a heart condition of clause 8, wherein the set of even waves and the set of odd waves are exclusively labeled as a set of A-waves or a set of V-waves.

10. The method of treating a heart condition of clause 9, wherein the exclusive labeling is performed using a machine learning model trained using a training data set comprising cleaned LAP signals annotated with A-waves and V-waves.

11. The method of treating a heart condition of clause 9, wherein the exclusive labeling is performed by waveform analysis comprising identification of double peaks in A-waves.

12. The method of treating a heart condition of clause 1, wherein the appropriate treatment comprises increasing a dosage of diuretics administered to the patient by between 40% and 60% in response to the A/V ratio dropping below 0.6.

13. The method of treating a heart condition of clause 1, wherein the appropriate treatment comprises increasing a dosage of hypertension medication while maintaining a dosage of diuretics to the patient in response to a mean blood pressure of the patient increasing above 40mmHg without a change in A/V ratio.

14. The method of treating a heart condition of clause 1, wherein the appropriate treatment is a drug which is provided via automatically delivered to the patient via an infusion pump.

15. A system for treating a heart condition of a patient, comprising:

a processor; and
a memory, the memory containing a cardiac monitoring application that configures the processor to:

obtain a left atrial pressure (LAP) signal recorded using an atrial cardiac sensor;
identify a set of A-waves and a set of V-waves in the LAP signal;
calculate an A/V ratio based on a mean pressure amplitude of the set of A-waves and a mean pressure amplitude of the set of V-waves; and
determine an appropriate treatment for the patient based on the A/V ratio.

16. The system for treating a heart condition of a patient of clause 15, wherein to identify the set of A-waves and the set of V-waves in the LAP signal, the cardiac monitoring application further configures the processor to decompose the LAP signal.

17. The system for treating a heart condition of a patient of clause 16, wherein the decomposition comprises application of a Fourier transform.

18. The system for treating a heart condition of a patient of clause 16, wherein the decomposition comprises application of a wavelet analysis.

19. The system for treating a heart condition of a patient of clause 16, wherein the cardiac monitoring application further configures the processor to determine a respiration rate of the patient by locating a dominant frequency between 0.1 and 0.5Hz in the decomposition

20. The system for treating a heart condition of a patient of clause 16, wherein the cardiac monitoring application further configures the processor to determine a heart rate of the patient by locating a dominant frequency between 0.8Hz and 1.7Hz in the decomposition.

21. The system for treating a heart condition of a patient of clause 16, wherein the cardiac monitoring application further configures the processor to subtract frequency components associated with respiration rate from the LAP signal to produce a cleaned LAP signal.

22. The system for treating a heart condition of a patient of clause 21, wherein the cardiac monitoring application further configures the processor to parse the cleaned LAP signal into a set of even waves and a set of odd waves.

23. The system for treating a heart condition of clause 22, wherein the set of even waves and the set of odd waves are exclusively labeled as a set of A-waves or a set of V-waves.

24. The system for treating a heart condition of clause 23, wherein the exclusive labeling is performed using a machine learning model trained using a training data set compri sing cleaned LAP signals annotated with A-waves and V-waves.

25. The system for treating a heart condition of clause 23, wherein the exclusive labeling is performed by waveform analysis comprising identification of double peaks in A-waves.

26. The system for treating a heart condition of clause 15, wherein the appropriate treatment comprises increasing a dosage of diuretics administered to the patient by between 40% and 60% in response to the A/V ratio dropping below 0.6; and wherein the cardiac monitoring application further configures the processor to deliver the dosage of diuretics via an infusion pump communicatively coupled to the processor.

27. The system for treating a heart condition of clause 15, wherein the appropriate treatment comprises increasing a dosage of hypertension medication while maintaining a dosage of diuretics to the patient in response to a mean blood pressure of the patient increasing above 40mmHg without a change in A/V ratio; and wherein the cardiac monitoring application further configures the processor to deliver the dosage of diuretics via an infusion pump communicatively coupled to the processor.

28. The system for treating a heart condition of clause 15, wherein the appropriate treatment is a drug which is provided via automatically delivered to the patient via an infusion pump communicatively coupled to the processor.

**Claims**

1. A cardiac patient exertion monitoring system, comprising:

   an atrial cardiac sensor configured to be implanted in a left atrium of a patient to record:

      a first blood pressure signal during a rest state of the patient; and

      a second blood pressure signal during an exertion state of the patient; and

   a cardiac signal processor configured to:

      receive the first and second blood pressure signals;
      calculate a first plurality of cardiac metrics based on the first blood pressure signal;
      calculate a second plurality of cardiac metrics based on the second blood pressure signals; and
      provide a warning indicating a health state of the patient based on a change between the first and second plurality of cardiac metrics.

2. The cardiac patient exertion monitoring system of claim 1, further comprising a transceiver configured to:

   obtain the first and second blood pressure signals from the atrial cardiac sensor; and
   transmit the first and second blood pressure signals to the cardiac signal processor.

3. The cardiac patient exertion monitoring system of claim 2, wherein the transceiver is further configured to power the atrial cardiac sensor.

4. The cardiac patient exertion monitoring system of any preceding claim, wherein powering the atrial cardiac sensor triggers the atrial cardiac sensor to record the first cardiac signal.

5. The cardiac patient exertion monitoring system of any preceding claim, further comprising an infusion pump; and wherein the cardiac signal processor is further configured to automatically deliver a drug to the patient via the infusion pump based on the health state of the patient.

6. The cardiac patient exertion monitoring system of any preceding claim, wherein the first and second blood pressure signals are left atrial pressure signals.

7. The cardiac patient exertion monitoring system of any preceding claim, wherein the first plurality of cardiac metrics and the second plurality of cardiac metrics comprise an A/V ratio.

8. The cardiac patient exertion monitoring system of any of claims 1 to 6, wherein the first plurality of cardiac metrics and the second plurality of cardiac metrics comprise a respiration rate.

9. The cardiac patient exertion monitoring system of any of claims 1 to 6, wherein the first plurality of cardiac metrics and the second plurality of cardiac metrics comprise a heart rate.

10. A process for exertion monitoring, comprising:

receiving, at a cardiac signal processor, a first blood pressure signal recorded by an atrial cardiac sensor implanted in a left atrium of a patient during a rest state of the patient;
receiving, at the cardiac signal processor, a second blood pressure signal recorded by an atrial cardiac sensor implanted in a left atrium of a patient during an exertion state of the patient;
calculating, using the cardiac signal processor, a first plurality of cardiac metrics based on the first blood pressure signal;
calculating, using the cardiac signal processor, a second plurality of cardiac metrics based on the second blood pressure signals; and
providing, using the cardiac signal processor, a warning indicating a health state of the patient based on a change between the first and second plurality of cardiac metrics.

11. The process of claim 10, further comprising:

obtaining, using a transceiver, the first and second blood pressure signals from the atrial cardiac sensor; and
transmitting, using the transceiver, the first and second blood pressure signals to the cardiac signal processor.

12. The process of claim 11, wherein the transceiver is further configured to power the atrial cardiac sensor.

13. The process of claim 12, wherein powering the atrial cardiac sensor triggers the atrial cardiac sensor to record the first cardiac signal.

14. The process of any of claims 10 to 13, wherein the first and second blood pressure signals are left atrial pressure signals.

15. The process of any of claims 10 to 14, wherein the first plurality of cardiac metrics and the second plurality of cardiac metrics comprise:

a) an A/V ratio;
b) a respiration rate; or
c) a heart rate.

*FIG. 1*

EP 4 736 757 A2

EP 4 736 757 A2

200

Processor

210

Input/Output Interface

220

Memory

Cardiac Monitoring Application

232

Cardiac Signal Data

234

Patient Data

236

230

*FIG. 2*

FIG. 3A

FIG. 3B

FIG. 4

FIG. 5

FIG. 6

Large Slow Respiration

*FIG. 7A*

Shallow Fast Respiration

*FIG. 7B*

*FIG. 8*

FIG. 9

FIG.10

EP 4 736 757 A2

EP 4 736 757 A2

FIG.11

FIG.12A

FIG.12B

FIG. 13A

FIG.13B

1400

Begin

1410 Obtain first cardiac signal at rest condition

1420 Generate first set of cardiac metrics

1430 Obtain second cardiac signal at exertion condition

1440 Generate second set of cardiac metrics

1450 Plot changes between the first and second sets of cardiac metrics

1460 Calculate ratios between first and second sets of cardiac metrics

End

*FIG.14*

FIG.15

*FIG.16*

*FIG. 17*

1800

Begin

1810 — Obtain patient data

1820 — Generate patient profile

1830 — Classify patient profile

1840 — Obtain cardiac signal

1850 — Calculate cardiac metrics

1860 — Display cardiac metrics in context of patient classification

End

*FIG.18*

FIG. 19

FIG.20

Mitral Valve Stenosis

Normal Mitral Valve

FIG. 21

*FIG.22*

2300

Begin

2310 — Obtain LAP signal

2320 — Calculate A/V Ratio

2330 — Identify local maximum and minimum for each A-wave and V-wave

2340 — Calculate average maximum and minimum pressure values across all A-waves and all V-waves

2350 — Calculate the difference between the A-wave maximum and the V-wave maximum

2360 — Calculate the difference between the A to V minimum and the V to A minimum

2370 — Calculate Mitral Valve Index

End

*FIG. 23*

FIG.24

EP 4 736 757 A2

*FIG.25*

FIG.26

FIG.27

*FIG. 28*

FIG. 29A

*FIG. 29B*

EP 4 736 757 A2

3000

Begin

3010 — Set hypertension level and pulmonary congestion alert level

3020 — Receive LAP Signal

3030 — Calculate daily mean LAP

3040 — Calculate moving average mean LAP

3050 — Calculate difference between moving average mean LAP and hypertension level when moving average mean LAP > hypertension level

3060 — Calculate pulmonary congestion risk index based on moving window of differences

3070 — Recommend treatment when pulmonary congestion risk index > pulmonary congestion alert level

End

*FIG. 30*

Mean Pressure

FIG. 31A

Mean Pressure

FIG. 31B

*FIG. 32*

*FIG. 33A*

Mean Pressure

FIG. 33B

EP 4 736 757 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63287003
- US 63287009
- US 63287016

- US 63291253
- US 63291270
- US 63291270 B